(19) Europäisches Patentamt
European Patent Office
Office européen des brevets

(11) **EP 1 774 032 B1**

(12) **FASCICULE DE BREVET EUROPEEN**

(45) Date de publication et mention
de la délivrance du brevet:
**21.04.2010 Bulletin 2010/16**

(21) Numéro de dépôt: **05775328.7**

(22) Date de dépôt: **07.06.2005**

(51) Int Cl.:
**C12Q 1/68** *(2006.01)*       **G06F 1/00** *(2006.01)*

(86) Numéro de dépôt international:
**PCT/FR2005/001392**

(87) Numéro de publication internationale:
**WO 2006/003298 (12.01.2006 Gazette 2006/02)**

(54) **PROCÉDÉ DE DÉTERMINATION DE LA CHARGE MUTATIONNELLE D'UNE BANQUE DE GÈNES OBTENUE PAR MUTAGÉNÈSE ALÉATOIRE D'UN GÈNE D'INTÉRÊT ET LES MOYENS POUR SA MISE EN OEUVRE**

VERFAHREN ZUR BESTIMMUNG DES MUTATIONSGRADES EINER DURCH ZUFÄLLIGE MUTAGENESE EINES BESTIMMTEN GENS GEWONNENE GENBIBLIOTHEK UND MITTEL ZU IHRER IMPLEMENTIERUNG

METHOD OF DETERMINING THE MUTATIONAL LOAD OF A GENE LIBRARY OBTAINED BY RANDOM MUTAGENESIS OF A PARTICULAR GENE AND MEANS FOR IMPLEMENTING SAME

(84) Etats contractants désignés:
**AT BE BG CH CY CZ DE DK EE ES FI FR GB GR HU IE IS IT LI LT LU MC NL PL PT RO SE SI SK TR**

(30) Priorité: **07.06.2004 FR 0406120**

(43) Date de publication de la demande:
**18.04.2007 Bulletin 2007/16**

(73) Titulaire: **Proteus**
**30000 Nîmes (FR)**

(72) Inventeurs:
• **CHODORGE, Matthieu**
**F-92160 Antony (FR)**
• **FOURAGE, Laurent**
**F-30870 Clarensac (FR)**
• **LEFEVRE, Fabrice**
**F-32120 Bajonnette (FR)**
• **MASSON, Jean-Michel**
**F-31400 Toulouse (FR)**

(74) Mandataire: **Novagraaf Technologies**
**122 rue Edouard Vaillant**
**92593 Levallois-Perret Cedex (FR)**

(56) Documents cités:
**WO-A-89/09272      DE-A- 19 818 422**

• **PATRICK WAYNE M ET AL: "User-friendly algorithms for estimating completeness and diversity in randomized protein-encoding libraries." PROTEIN ENGINEERING, vol. 16, no. 6, juin 2003 (2003-06), pages 451-457, XP002312826 ISSN: 0269-2139**
• **GREENER A ET AL: "AN EFFICIENT RANDOM MUTAGENESIS TECHNIQUE USING AN E.COLI MUTATOR STRAIN" MOLECULAR BIOTECHNOLOGY, TOTOWA, NJ, US, vol. 7, no. 2, avril 1997 (1997-04), pages 189-195, XP000974365 ISSN: 1073-6085**
• **STEFAN A ET AL: "Directed evolution of beta-galactosidase from Escherichia coli by mutator strains defective in the 3'->5' exonuclease activity of DNA polymerase III" FEBS LETTERS, ELSEVIER SCIENCE PUBLISHERS, AMSTERDAM, NL, vol. 493, no. 2-3, 30 mars 2001 (2001-03-30), pages 139-143, XP004257419 ISSN: 0014-5793**

**Description**

La présente invention se rapporte à un procédé permettant de déterminer la charge mutationnelle introduite dans une banque de gènes à l'issue d'une étape de mutagenèse aléatoire d'un gène d'intérêt. L'invention se rapporte également aux moyens pour la mise en oeuvre de ce procédé comme des kits et des supports destinés à être exploités par des ordinateurs.

La mutagenèse aléatoire des acides nucléiques permet d'introduire des modifications dans les séquences d'acides aminés des protéines. Il s'agit d'une stratégie alternative performante à la mutagenèse dirigée et au design rationnel des protéines. En effet, en l'absence de toutes données précises sur les caractéristiques d'une protéine comme sa structure, c'est à dire dans la majorité des cas, seule la mutagenèse aléatoire permet d'identifier rapidement des mutants ou des variants d'un gène ayant acquis de nouvelles propriétés.

De manière générale, bien qu'il ait été possible d'isoler des variants améliorés dans des banques hyper-mutées (Zaccolo M. et al. (1999) Journal of Molecular Biology. 285 :775-783), il est admis que l'introduction d'un trop grand nombre de mutations conduit à une majorité de protéines inactives et limite sévèrement l'identification de variants améliorés alors qu'un taux de mutation trop faible diminue la probabilité d'obtenir une mutation bénéfique. Ainsi plusieurs exemples d'évolution dirigée tendraient à montrer que le taux de mutation le plus efficace pour identifier des variants améliorés serait de 1-5 acides aminés par protéine, soit 2-7 nucléotides par gène (Cherry J .R. et al.. (1999) Nature Biotechnology. 17 :379-384. / Shafikhani S. et al. (1997) BioTechniquers 23 :304-310. / Moore J.C. et al. (1997) Journal of Molecular Biology. 272 :336-347.)

Il est donc nécessaire de pouvoir contrôler le nombre de mutations introduites dans une banque de gènes par mutagenèse aléatoire. La charge mutationnelle (CM) est définie comme le nombre moyen de mutations par gène au sein d'une banque de gènes mutés.

La méthode de référence de l'art antérieur pour déterminer précisément le taux de mutation dans une banque de gènes mutés reste aujourd'hui l'observation statistique par séquençage d'une fraction de gènes de la banque. Cette méthode reste encore lourde à mettre en oeuvre et surtout est très coûteuse.

Comme alternative au séquençage, d'autres méthodes pour quantifier le taux de mutation ont été développées comme par exemple le « Single Strand Conformation Polymorphism » (SSCP) ou le « Denaturing Gradient Gel Electrophoresis » (DGGE) qui permettent de détecter des différences entre les séquences jusqu'à une base (Cariello et al. (1993) Mutational Research 288 :103-112 / Brail et al, (1993) Mutational Research 303 :171-175). Néanmoins ces techniques sont plutôt adaptées à une analyse qualitative comme la détection de mutation et la détermination de faibles taux d'erreur qu'à une quantification de charge mutationnelle.

Une autre méthode de l'art antérieur fait appel à l'analyse des changements dans le profil de restriction de séquences ayant intégrées ou perdues des sites de restriction particuliers. Cette méthode s'applique donc uniquement à des séquences particulières avec une modification de sites de restriction représentatifs.

Il a aussi été proposé une méthode de suivi de la fréquence de mutation basée sur des tests phénotypiques où un gène rapporteur est soumis à une mutagenèse aléatoire avant clonage. Les fréquences de mutations sont alors simplement analysées en enregistrant la perte de fonctionnalité du produit du gène rapporteur due à l'apparition de mutations dans celui-ci. Cette dernière méthode est cependant limitée à des gènes pour lesquels l'introduction de mutations peut être observée facilement par un test phénotypique Greener et al., Mol. Biotech. 7, 189-195 (1997). Il est également décrit dans l'art antérieur des tables permettant d'associer à des conditions expérimentales une charge mutationnelle d'une banque de gènes mutés pour un gène d'intérêt donné en fonction de sa longueur. Ces tables sont construites sur la base d'un gène modèle soumis à différentes conditions expérimentales puis par le séquençage d'un nombre statistiquement représentatif de gènes de la banque de gènes mutés. Cependant, ces techniques ne tiennent compte que de la longueur dudit gène d'intérêt (Gene Morph®II Random Mutagenesis kit, Stratagene).

De plus, aucune des méthodes de l'art antérieur n'utilisent de standard interne permettant de tenir compte des variabilités expérimentales susceptibles d'influencer la charge mutationnelle d'une banque. Ce qui signifie que ces méthodes ne sont pas suffisamment fiables et reproductibles pour qu'il soit possible de s'affranchir du séquençage afin de déterminer la charge mutationnelle obtenue dans une banque de gènes mutés.

Dans le cas des méthodes de mutagénèse aléatoire *in vivo,* on peut \supposer que la variabilité expérimentale est plus faible. Cependant, leur application est limitée aux faibles charges mutationnelles (1 à 2 mutations par Kb) et ne permet pas d'envisager des chargements de séquence multiples (Greener et al, 1997) car elles nécessiteraient un nombre de générations impossible à mettre en oeuvre d'un point de vue pratique.

La présente invention a précisément pour but d'offrir dans le domaine de la mutagenèse aléatoire des méthodes fiables pour la prédiction de la charge mutationnelle de banques de gènes mutés.

Ce but est atteint selon l'invention grâce à un procédé de détermination de la charge mutationnelle d'une banque de gènes obtenue par mutagenèse aléatoire d'un gène d'intérêt, **caractérisé en ce qu**'il comprend les étapes suivantes :

a) la préparation d'un abaque liant la charge mutationnelle (CM) d'une banque de gènes obtenue par mutagenèse aléatoire d'un gène modèle à la fraction des gènes modèles mutés observés dans ladite banque ;

b) la mutagenèse aléatoire du gène modèle ayant servi pour la préparation de l'abaque de l'étape (a) et du gène d'intérêt pour obtenir les banques de gènes mutés correspondantes ;

c) la détermination de la charge mutationnelle (CM) de la banque de gènes obtenue à partir du gène modèle à l'étape (b) à partir de l'abaque établi à l'étape (a) ;

d) l'application d'un facteur de correction (CF) à la charge mutationnelle (CM) de la banque de gènes modèles mutés déterminée à l'étape (c) pour déterminer la charge mutationnelle (CM) de la banque de gènes d'intérêt mutés de l'étape (b).

Selon une forme toute préférée du procédé de l'invention, la technique de mutagenèse mise en oeuvre à l'étape (b) est identique pour le gène modèle et pour le gène d'intérêt et est également similaire à celle de l'étape (a) pour construire l'abaque à partir du gène modèle. Avantageusement, on entend par technique identique ou similaire, le fait que non seulement la technique est la même mais aussi les conditions de mise en oeuvre. La similarité ou l'identité de la technique de mutagenèse et de ses conditions n'excluent pas le fait que l'homme de métier puisse y apporter des aménagements qui ne soient pas de nature à modifier substantiellement les performances de la technique de mutagenèse. A titre d'exemple, les polymérases utilisées aux étapes (a) et (b) pour mettre en oeuvre une technique de PCR error-prone peuvent ne pas être identiques.

De même, l'homme de métier est à même de déterminer un gène modèle pour l'étape b) différent de celui de l'étape a), mais présentant des propriétés substantiellement équivalentes pour la mise en oeuvre desdites étapes.

Le procédé de l'invention est remarquable en ce qu'il offre une précision suffisante pour constituer une alternative efficace au séquençage qui est très coûteux. Ainsi les étapes (c) et (d) peuvent s'affranchir du séquençage.

Dans la suite de la description de l'invention qui suit, on utilisera indifféremment :

« banque de gènes obtenue par mutagenèse aléatoire d'un gène modèle » ou « banque de gènes modèles mutés » ; et

« banque de gènes obtenue par mutagenèse aléatoire d'un gène d'intérêt » ou « banque de gènes d'intérêt mutés »

On entend par gène au sens de la présente invention, toute séquence d'acide nucléotidique, codante ou non.

Une caractéristique essentielle de la présente invention réside dans le fait que les modifications de la séquence du gène modèle peuvent être observées après mutagenèse.

On entend par gènes modèles mutés les gènes modèles dont la séquence nucléotidique est modifiée par rapport à celle du gène modèle avant mutagenèse.

On entend par gène modèle muté observé un gène modèle dont la modification de séquence est observable.

On entend par observable le fait que l'introduction aléatoire de mutation n'est pas analysée par séquençage mais par l'analyse directe ou indirecte de la modification d'une ou plusieurs propriétés des gènes modèles mutés par rapport au gène modèle avant mutagenèse.

On entend par analyse indirecte le fait que la modification d'une ou plusieurs propriétés du gène muté est observée par une réaction intermédiaire.

A titre d'exemple la modification d'un gène modèle peut être observée par la modification d'une ou plusieurs propriétés de la protéine codée par le gène modèle. La modification d'une ou plusieurs propriétés de la protéine peut consister en la perte ou le gain d'activité(s) caractérisant la protéine.

Dans une forme préférée de réalisation de l'invention, l'observation des gènes modèles mutés est réalisée par un test phénotypique. On entend par test phénotypique, un test permettant de détecter la fonction de la protéine codée par le gène modèle, muté ou non.

A titre d'exemple de gène dont la fonction peut être détectée par un test phénotypique, on peut citer un gène :

- impliqué dans la résistances aux antibiotiques,
- impliqué dans l'auxotrophie de la souche hôte,
- codant pour une protéine présentant une activité aisément détectable (beta-galactosidase xylanase / Green Fluorescent Protein (GFP...),
- codant pour une protéine impliquée dans une réaction immunologique de type antigène-anticorps.

Le gène modèle est utilisé dans la méthode de l'invention pour :

- établir un abaque tel que défini dans l'étape (a), et
- être utilisé en parallèle du gène d'intérêt à l'étape (b) pour tenir compte des variabilités que peuvent avoir les conditions expérimentales sur les résultats de mutagenèse aléatoire, et ainsi servir de standard interne.

Contrairement à un témoin classiquement utilisé en biologie, le standard interne défini dans le procédé de l'invention par le gène modèle, n'indique pas uniquement le bon déroulement ou le degré d'avancement d'une expérience, il donne en plus une mesure nécessaire à la réalisation du procédé.

Dans une forme particulière de réalisation de l'invention, le gène modèle est un gène modèle dit universel, c'est à dire, qu'il possède des caractéristiques partagées par un grand nombre de gènes. A titre d'exemple, il présente une longueur comprise entre 500pb et 10kpb et un pourcentage en GC compris entre 20 et 80. Préférentiellement, le gène modèle universel présente un pourcentage en GC compris entre 40 et 60. A titre d'exemple d'un gène modèle universel, on peut citer le gène codant pour la protéine GFP, dont l'introduction de mutations peut être réalisée par un test phénotypique selon lequel la fluorescence des colonies sous ultraviolet reflète un phénotype GFP actif.

Dans une autre forme préférée de réalisation de l'invention, le gène modèle présente des caractéristiques proches de celles du gène d'intérêt. Ces caractéristiques correspondent avantageusement à des propriétés intrinsèques. Il est entendu par propriété intrinsèque d'un gène des propriétés inhérentes à la séquence de ce gène.

Parmi les propriétés intrinsèques d'un gène envisagées ci-dessus, on peut citer à titre d'exemple et de manière non limitative :

- sa longueur,
- sa composition en base, la composition en base peut être avantageusement analysée sur un seul brin.
- sa fréquence de bases répétées dans la séquence,
- la présence de séquences symétriques dans le gène.

Une fois le gène modèle sélectionné, une technique de mutagenèse aléatoire est choisie pour mettre en oeuvre la méthode de l'invention. Il est entendu par technique de mutagenèse aléatoire toute technique connue de l'homme de métier. Elle peut correspondre également à une combinaison de plusieurs techniques de mutagenèse. Elle peut être *in vivo* ou *in vitro*.

Dans une forme de mise en oeuvre préférée, la méthode de l'invention se rapporte à des techniques de mutagenèse *in vitro* basée préférentiellement sur l'utilisation d'une polymérase ou sur la mise en oeuvre d'une technique d'amplification. A titre d'exemple, la technique de mutagenèse correspond à la PCR error-prone (Cadwell R.C et al. (1992) PCR Methods Appl. 2 :28-33. / Leung D.W. et al. (1989) Techniques 1 :11-15.)

Une fois le gène modèle sélectionné et la technique de mutagenèse choisie, un abaque permettant de lier la charge mutationnelle d'une banque de gènes obtenue par mutagenèse aléatoire d'un gène modèle à la fraction de gènes modèles mutés observés dans ladite banque est préparé (étape a).

Il est entendu par abaque au sens de l'invention tout graphique, toute table ou toute équation permettant de lier de manière biunivoque la charge mutationnelle (CM) d'une banque de gènes obtenue par mutagenèse aléatoire d'un gène modèle à la fraction de gènes modèles mutés observés dans ladite banque.

Un abaque est obtenu pour une technique de mutagenèse et un gène modèle donné.

Ainsi, dans la forme préférée de l'invention où la modification de la séquence du gène modèle est observable par un test phénotypique, l'abaque obtenu à l'étape (a) permet de lier la charge mutationnelle d'une banque de gènes obtenue par mutagenèse aléatoire du gène modèle à la fraction de gènes présentant un phénotype mutant dans ladite banque.

L'abaque peut avoir été réalisé au préalable par l'homme de l'art ou peut être obtenu de la manière suivante :

i) Une technique de mutagenèse donnée est appliquée à un gène modèle préalablement sélectionné pour obtenir une banque de gènes modèles mutés.
ii) La charge mutationnelle (CM) dans la banque de gènes modèles mutés est ensuite déterminée par séquençage. Il est observé en parallèle la fraction de gènes modèles mutés dans ladite banque présentant une modification d'une ou plusieurs propriétés par rapport au gène sauvage.

Dans une forme préférée de l'invention, un nombre de clones statistiquement représentatif est analysé et le gène modèle muté correspondant est séquencé. Dans une forme préférée de l'invention, entre 10 et 100 clones sont séquencés. Avantageusement, le séquençage est effectué sur 25 clones tandis que l'analyse de la fraction de clones présentant une modification de séquence est effectuée sur au moins 500 clones.

Une liaison biunivoque peut ainsi être établie entre la charge mutationnelle d'une banque de gènes obtenue par mutagenèse aléatoire d'un gène modèle et la fraction de gènes modèles mutés observés dans ladite banque.

Disposant de cet abaque, la technique de mutagenèse aléatoire utilisée pour construire l'abaque est appliquée en parallèle au gène modèle et à un gène d'intérêt à l'étape (b), le gène modèle de l'étape (b) étant de préférence le même que celui ayant servi à construire l'abaque à l'étape (a).

A l'étape (b), la même technique de mutagenèse est appliquée simultanément au gène modèle et au gène d'intérêt.

Le fait d'utiliser le gène modèle avec le gène d'intérêt à l'étape (b) revient à utiliser le gène modèle comme standard interne. Le fait d'utiliser le gène modèle comme standard interne présente l'avantage :

- de tenir compte des variations expérimentales qui peuvent avoir lieu lors d'une expérience effectuée à l'étape (b) sur le gène modèle par rapport à celles effectuées antérieurement pour obtenir la relation définie à l'étape (a) ;
- de minimiser les variations expérimentales d'un échantillon à un autre, c'est à dire du traitement du gène modèle par rapport au gène d'intérêt lors de l'étape (b).

Ces variations expérimentales sont non négligeables sur la charge mutationnelle observée. En effet, il a été démontré que des modifications même minimes des conditions expérimentales peuvent avoir des conséquences importantes sur la charge mutationnelle introduite dans une banque. Le tableau 1 ci-après présente les moyennes, les écart-types et les coefficients de variations C.V. (C.V.=écart-type/moyenne en %) des fractions de clones exprimant une GFP fonctionnelle à l'issue de six expériences répétées de PCR error-prone effectuées avec différentes concentrations de $MgCl_2$ et $MnCl_2$ (paramètres de contrôle du niveau de mutagenèse). A partir d'une expérience répétée à l'identique, le résultat de la mutagenèse error-prone est fortement variable avec des coefficients de variations sur les fractions de clones exprimant une GFP fonctionnelle, et donc du taux de mutation compris entre 10 et 40%.

Tableau 1

| Conditions réactionelles | | Fraction population clones *gfp* active | | |
|---|---|---|---|---|
| [MgCl2] mM | [MnCl2] mM | Moyenne | Ecart Type | CV(%) |
| 5 | 0 | 0,722 | 0,071 | 9,9 |
| 7 | 0,2 | 0,411 | 0,122 | 29,7 |
| 7 | 0,4 | 0,091 | 0,040 | 43,8 |
| 7 | 0,5 | 0,036 | 0,013 | 35,5 |

Le tableau 1 ci-dessus indique la variabilité de la fraction de clones exprimant une GFP fonctionnelle dans des banques de gènes *gfp* mutés aléatoirement par PCR error-prone lors d'expériences répétées 6 fois à l'identique.

L'étape (b) est donc l'étape clé de la méthode de l'invention. Elle permet de tenir compte des variations expérimentales pouvant avoir lieu lors de la mise en oeuvre d'une technique de mutagenèse aléatoire sur un gène d'intérêt, lesdites variations pouvant influencer la définition de la charge mutationnelle de la banque de gènes ainsi obtenue. De ce fait, la méthode de l'invention permet de s'affranchir du séquençage au niveau des étapes (c) et (d) pour déterminer la charge mutationnelle d'une banque de gènes d'intérêt mutés.

Dans une forme toute préférée de l'invention, le même mélange réactionnel est utilisé pour mettre en oeuvre en parallèle la technique de mutagenèse sur le gène modèle et sur le gène d'intérêt pour minimiser encore les variations expérimentales d'un échantillon à un autre.

La banque de gènes modèles mutés est ensuite analysée pour déterminer la charge mutationnelle de la banque correspondante (étape c). A l'issue de l'étape (b), la fraction de gènes modèles mutés observés dans la banque obtenue après mutagenèse du gène modèle est déterminée. Grâce à l'abaque défini à l'étape (a), la fraction de gènes modèles mutés observés dans ladite banque permet de définir la charge mutationnelle de cette banque.

A l'étape (d), la charge mutationnelle déterminée à l'étape (c) est corrigée en lui appliquant un facteur de correction (CF) pour déterminer la charge mutationnelle introduite dans la banque de gènes obtenue par mutagenèse aléatoire du gène d'intérêt.

Ceci est réalisé selon l'équation suivante :

$$\text{CM (gène d'intérêt) = CM (gène modèle) x CF}$$

dans laquelle, CF représente le facteur de correction, et CM représente la charge mutationnelle.

Le facteur de correction (CF) peut se décomposer en plusieurs sous-facteurs de correction suivant les paramètres sélectionnés comme influençant la charge mutationnelle d'une banque de gènes d'intérêt mutés par rapport à une banque de gènes modèle mutés.

Un sous-facteur de correction permet de normaliser l'effet d'un paramètre sélectionné comme capable d'influencer la charge mutationnelle d'une banque par rapport à une autre.

La construction du facteur de correction CF s'effectue par la multiplication des différents sous-facteurs que l'on souhaite prendre en compte pour la détermination de la charge mutationnelle de la banque de gènes d'intérêt mutés dans la méthode de l'invention.

Les paramètres sélectionnés comme pouvant influencer la charge mutationnelle peuvent correspondre à titre d'exemple à un ou plusieurs des paramètres suivants :

- paramètres intrinsèques
- paramètres expérimentaux.

Il est entendu par paramètres intrinsèques d'un gène ceux qui découlent de la séquence de ce gène comme indiqué précédemment.

Il est entendu par paramètre expérimental tout paramètre capable d'engendrer des différences dans l'introduction de mutations dans le gène modèle par rapport au gène d'intérêt lors de la mise en oeuvre de la technique de mutagenèse.

La nature de ces paramètres expérimentaux sont dépendants de la technique de mutagenèse aléatoire utilisée. Ils ne peuvent être calculés qu'après avoir mis en oeuvre la technique de mutagenèse sur le gène modèle et sur le gène d'intérêt à l'étape (b).

La considération de ce type de paramètre pour effectuer la correction à l'étape (d) s'ajoute à l'avantage d'utiliser un standard interne afin de tenir compte au mieux des variations expérimentales sur la détermination de la charge mutationnelle d'une banque de gènes.

Lors de techniques de mutagenèse basées sur l'usage de la PCR (dont la PCR error-prone), les paramètres expérimentaux peuvent correspondre, de manière non limitative :

- au nombre de multiplication du gène durant l'étape de mutagenèse aléatoire,
- à la quantité de matrice initiale,
- au nombre de cycle d'amplification,
- à la nature et à la quantité de polymérase utilisée
- aux conditions expérimentales utilisées comme la concentration en cations divalents, la concentration et le ratio de concentration des dNTPs.

La définition des différents sous-facteurs de correction doit s'effectuer suivant des lois mathématiques prenant en compte la nature des évènements biologiques qui seront normalisés.

Par exemple, il est convenu dans l'art antérieur que le nombre de mutations introduites dans un gène est lié linéairement à la longueur du gène (paramètre intrinsèque).

Ainsi CF[L], défini comme le sous-facteur de correction lié à la longueur des gènes, est explicité comme suit :

$$\texttt{CF[L] = L(gène d'intérêt) / L(gène modèle)}$$

où L correspond à la longueur des gènes.

De même, il est connu de l'art antérieur que les différentes méthodes de mutagenèse n'affectent pas de manière équivalente les différents nucléotides A, T, G ou C. Pour chaque méthode de mutagenèse on peut définir un sous-facteur de correction CF[base] qui tient compte des différences de composition en bases entre le gène modèle et le gène d'intérêt vis à vis du biais d'introduction de mutation de la technique de mutagenèse.

Pour établir un tel sous-facteur de correction il est nécessaire d'introduire la notion de matrice cible des mutations.

En effet, la matrice cible des mutations du gène modèle (Mtarget[X] où X représente le gène modèle) décomposent sur les quatre bases la probabilité de toucher un type de base particulier dans le gène modèle et permet de normaliser la composition en base par rapport au gène modèle utilisé.

La matrice cible des mutations du gène modèle est déduite à partir de séquençages des gènes modèles mutés par une technique définie de mutagenèse aléatoire, et identification des mutations apparues.

L'analyse des mutations apparues dans les séquences des gènes modèles mutés permet d'établir la matrice des mutations $Mut_x$, où $Mut_x$ et répertorie les fréquences des 12 types possibles de mutations, selon la matrice suivante :

$$Mut_x = \begin{pmatrix} & f_{A \to T} & f_{A \to C} & f_{A \to G} \\ f_{T \to A} & & f_{T \to C} & f_{A \to G} \\ f_{C \to A} & f_{C \to T} & & f_{C \to G} \\ f_{G \to A} & f_{G \to T} & f_{G \to C} & \end{pmatrix}$$

dans laquelle, $f_{X \to Y}$ représente la probabilité de muter la base X en base Y.

La matrice cible de mutation du gène modèle X, $M_{target}[X]$ est alors déduite de la matrice $Mut_x$ en sommant les trois fréquences affectant un type de base selon la matrice suivante :

$$M_{target}[X] = \begin{pmatrix} A_{Mtarg[X]} \\ T_{Mtarg[X]} \\ C_{Mtarg[X]} \\ G_{Mtarg[X]} \end{pmatrix}$$

dans laquelle, $\displaystyle Z_{Mtarg[X]} = \sum_{Y-(A,T,C,G)-Z} f_{Z \to Y}$ , où Z est A, T, C ou G.

La matrice cible des mutations est dépendante de la nature du gène modèle. Plus un gène modèle sera riche dans un type de base particulier plus la probabilité d'affecter ce type de base avec une méthode définie de mutagenèse aléatoire sera élevée.

La matrice cible des mutations est dépendante de la technique de mutagenèse utilisée car cette dernière est fonction du biais de la méthode de mutagenèse aléatoire.

Ainsi CF[base], défini comme le sous-facteur de correction lié à la composition en base, est explicité selon l'équation suivante :

$$C.F.[base] = \left( \frac{A_{Mtarg[X]} \times A_{(g.interêt)}}{A_{(X)}} + \frac{T_{Mtarg[X]} \times T_{(g.interêt)}}{T_{(X)}} + \frac{C_{Mtarg[X]} \times C_{(g.interêt)}}{C_{(X)}} + \frac{G_{Mtarg[X]} \times G_{(g.interêt)}}{G_{(X)}} \right)$$

dans laquelle, $A_{(g.interêt)}$, $A_{(x)}$, $T_{(g.interêt)}$, $T_{(x)}$, $C_{(g.interêt)}$, $C_{(x)}$ $G_{(g.interêt)}$ et $G_{(x)}$ correspondent respectivement à la composition en base A, T, C et G du gène modèle X et du gène d'intérêt.

De plus, l'impact de certains mutagènes sur un nucléotide donné peut varier significativement selon le type de nucléotide qui lui est adjacent dans la séquence. C'est pourquoi le facteur correctif pourra intégrer, lorsque cela sera pertinent, un terme de comparaison entre les fréquences des dinucléotides dans le gène d'intérêt par rapport au gène modèle.

La fréquence relative des trinucléotides entre gène modèle et gène d'intérêt pourra de même être prise en compte dans le facteur correctif.

A titre d'exemple également, dans le cas d'une mutagenèse «*in vitro*» basée sur la PCR, le taux de multiplication (paramètre expérimental) subit par la matrice va directement influencer le taux de mutation. En effet, l'efficacité de réplication est dépendante de la matrice ADN et du programme PCR utilisé. Dues à des différences de longueur, de composition en base, de la présence de structure secondaire, certains gènes sont plus difficiles à amplifier que d'autres. Ainsi, les éventuelles différences de rendement PCR entre la mutagenèse du gène modèle et celle du gène d'intérêt pourront générer des différences dans le taux de multiplication et ceci même en partant d'une quantité identique de matrice et en réalisant un même nombre de cycle PCR. Le taux de multiplication (d) est calculé d'après l'équation suivante :

```
d = ln(Qf/Qi)/ln2
```

dans laquelle, Qi et Qf représentent respectivement les quantités molaires de matrice initiale et de produit à l'issue de la PCR.

Les quantités initiale de matrice et finale de produit peuvent être déterminées par de nombreux moyens connus de l'homme de métier. Citons par exemple le dosage spectrophotométrique aux UV ou le calibrage par rapport à une quantité connue d'ADN.

Les paramètres expérimentaux peuvent également correspondre au nombre de générations d'une culture cellulaire lors d'une technique de mutagenèse « in vivo ». A titre d'exemple, dans le cas d'une mutagenèse «in vivo», le nombre de mutations introduites dans les gènes va dépendre du nombre de générations de la culture. L'expérimentateur doit alors prendre soin de l'analyser, par exemple en mesurant la turbidité des cultures.

Différentes formes de réalisation de l'invention peuvent être envisagées, où :

- au moins un paramètre intrinsèque de chacun des gènes modèle et d'intérêt est considéré pour calculer le facteur de correction,
- au moins deux paramètres intrinsèques du gène modèle et du gène d'intérêt sont considérés pour calculer le facteur de correction selon l'équation suivante :

$$CF = CF[\text{ paramètre intrinsèque 1}] \times CF[\text{ paramètre intrinsèque 2}]$$

A titre d'exemple, ces paramètres intrinsèques correspondent à la longueur et à la composition en base des gènes, selon l'équation suivante :

$$CF = CF[L] \times CF[\text{base}]$$

dans laquelle :

- CF[L] , le sous-facteur de correction est lié à la longueur des gènes,
- CF[base], le sous-facteur de correction est lié à la composition en base des gènes,
- au moins un paramètre intrinsèque et au moins un paramètre expérimental sont considérés pour établir le facteur de correction, avec C.F. déterminé selon l'équation suivante :

$$CF = CF[\text{paramètre intrinsèque}] \times CF[\text{paramètre expérimental}]$$

- au moins deux paramètres intrinsèques et au moins un paramètre expérimental sont considérés pour établir le facteur de correction, avec CF déterminé selon l'équation suivante :

$$CF = CF[\text{paramètre intrinsèque 1}] \times CF[\text{paramètre intrinsèque 2}] \times CF[\text{paramètre expérimental}]$$

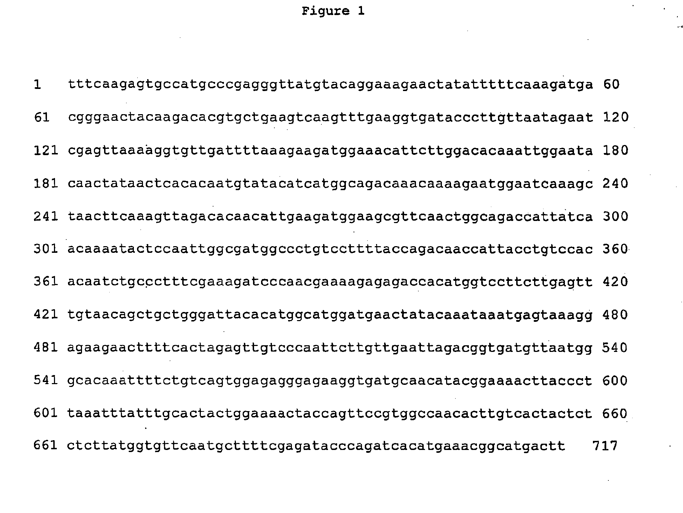

Dans le cas particulier où la technique de mutagenèse correspond à la PCR error-prone et le gène modèle correspond à la GFP (séquence de la Figure 1), différents sous-facteurs de correction correspondent pour :

- paramètre intrinsèque 1 représente la longueur des gènes selon l'équation suivante :

$$CF[L] = L(\text{gène d'intérêt})/717$$

où L est en nombre de paire de base,

- paramètre intrinsèque 2 représente la composition en base sur les brins d'ADN selon l'équation suivante :

$$C.F.[base] = \left( \frac{0{,}506 \times A_{(g.interêt)}}{0{,}34} + \frac{0{,}346 \times T_{(g.interêt)}}{0{,}26} + \frac{0{,}063 \times C_{(g.interêt)}}{0{,}197} + \frac{0{,}085 \times G_{(g.interêt)}}{0{,}203} \right)$$

où $A_{(g.interêt)}$, $T_{(g.interêt)}$, $C_{(g.interêt)}$ et $G_{(g.interêt)}$ sont en fraction,

- paramètre expérimental représente le taux de multiplication selon l'équation suivante :

$$CF[d] = d(\text{gène d'intérêt}) / d(gfp)$$

où d est le taux de multiplication calculé selon l'équation suivante :

$$d = \ln(Qf/Qi)/\ln 2$$

dans laquelle, Qi et Qf représentent respectivement les quantités molaires de la matrice initiale et des produits à l'issue de la PCR.

L'invention concerne aussi les utilisations du procédé préalablement défini :

1) Le procédé de l'invention peut être utilisé pour obtenir et contrôler la charge mutationnelle désirée dans une banque de gènes d'intérêt mutés. L'expérimentateur, sur la base d'un abaque lui permettant de lier la charge mutationnelle d'une banque de gènes modèles mutés aux conditions expérimentales de mutagenèse aléatoire, va sélectionner les conditions expérimentales ou une gamme de conditions expérimentales lui permettant de se rapprocher d'une charge mutationnelle désirée pour sa banque obtenue après mutagenèse aléatoire d'un gène d'intérêt. L'expérimentateur va faire cette sélection en tenant compte au moins d'un paramètre intrinsèque des gènes modèles et d'intérêt. L'expérimentateur met alors en oeuvre les étapes (a) à (d) du procédé de l'invention préalablement décrite en utilisant à l'étape (b) les conditions expérimentales préalablement sélectionnées. Il utilise alors la méthode de l'invention pour vérifier que la charge mutationnelle introduite est bien celle désirée. Ainsi, l'utilisation de la méthode de l'invention pour obtenir et contrôler une charge mutationnelle désirée dans une banque de gènes d'intérêt mutés est caractérisée par la mise en oeuvre d'une étape supplémentaire de sélection des conditions expérimentales de mutagenèse aléatoire qui seront utilisées pour mettre en oeuvre l'étape (b). A titre d'exemple cette utilisation permet de contrôler la charge mutationnelle dans une banque de gènes d'intérêt mutés en préalable à une étude structure-fonction. L'expérimentateur réalise premièrement une banque de gènes d'intérêt faiblement muté puis il vérifie, par la méthode de l'invention, que la charge mutationnelle introduite est bien de 1-2 mutations par gène, le nombre de mutation optimale pour ce type d'application (Vartanian J.P., Henry M., Wain-Hobson S. Hypermutagenic PCR involving all four transitions and a sizeable proportion of transversions. Nucleic Acids Res. (1996). 24 :2627-2631). Le criblage des variants issus de la banque dans une fonction donnée et le séquençage des clones identifiés positifs permettent ensuite de lier sans ambiguïté la séquence à la fonction étudiée.

2) Le procédé de l'invention peut être utilisé pour contrôler la charge mutationnelle dans une banque de gènes d'intérêt mutés en préalable à une recherche de variants améliorés. L'expérimentateur réalise premièrement une banque de gène d'intérêt muté puis il vérifie, par la méthode de l'invention, que la charge mutationnelle introduite est bien de 3-7 mutations par gène, le nombre de mutations optimale pour ce type d'application (Wan L. et al. (1998) Proc. Natl. Acad. Sci. USA 95 :12825-12831. / You L et al. (1996)Protein Eng. 9 :77-83). L'application d'un nombre optimal de mutation permet d'augmenter la probabilité d'identifier des gènes codant pour des protéines améliorées.

3) Le procédé de l'invention peut être utilisé pour étudier la plasticité protéique c'est à dire la capacité à incorporer des mutations tout en conservant une activité présentée par la protéine sauvage (Shafikhani S. et al. (1997) Bio-Techniquers 23 :304-310). L'expérimentateur réalise premièrement des banques de gènes d'intérêt plus ou moins mutés puis il détermine, par la méthode de l'invention, les charges mutationnelles associées aux différentes banques. L'analyse des fractions de clones produisant un variant protéique fonctionnel dans les différentes banques permet l'étude de la plasticité du produit du gène d'intérêt en liant ces dernières aux charges mutationnelles précédemment déterminées.

4) Le procédé de l'invention peut être utilisé pour étudier le taux d'évolution d'une protéine d'intérêt, c'est à dire la capacité à acquérir une nouvelle propriété avec une charge mutationnelle donnée (Miura T. et al. (2001) J. theor. Biol. 209 :497-502). L'expérimentateur réalise premièrement des banques de gènes d'intérêt plus ou moins mutés puis il détermine, par la méthode de l'invention, les charges mutationnelles associées aux différentes banques. L'analyse des fractions de clones produisant un variant présentant une propriété originale par rapport à la protéine sauvage dans les différentes banques permet d'étudier le taux d'évolution en fonction de la charge mutationnelle.

5) L'invention peut être utilisée pour déterminer la charge mutationnelle optimale dans des banques de gènes d'intérêt mutés pour générer un lot de gènes parentaux avant la mise en oeuvre de méthodes de recombinaison moléculaire. L'expérimentateur réalise premièrement des banques de gènes d'intérêt plus ou moins mutés puis il détermine, par la méthode de l'invention, les charges mutationnelles associées aux différentes banques. Deuxièmement la réalisation de criblages adéquats sur les banques de gènes d'intérêt mutés permettent d'identifier des clones présentant une/des propriété(s) améliorée(s) par rapport à celles présentées par la protéine sauvage. Chacune des banques de gènes d'intérêt mutés génèrent ainsi un lot de gènes optimisés, qui servent alors de gènes parentaux dans une étape de recombinaison. Les différentes banques de gènes recombinés sont criblées pour identifier des protéines encore plus améliorées que celles produites par les gènes parentaux ou par le gène sauvage. L'analyse et la comparaison de ces banques, par exemple sur la fréquence d'apparition de clones améliorés ou sur la quantification des améliorations obtenues, permettent de déterminer la charge mutationnelle optimale à introduire initialement dans une banque de gène d'intérêt en préambule à une étape de recombinaison. Toutes les méthodes de recombinaison *in vitro* : DNA Shuffling (Stemmer W. (1994) Nature 370 :389-391), L-Shuffling (WO0009679), StEP (Zhao H. et al. (1998) Nature Biotechnology 16 :258-261), RACHiTT (Coco W. et al. (2001) Nature Biotechnology 19 :354-359) et *in vivo* comme la recombinaison dans la levure (Pompon D. et al. (1989) Gene 83:15-24), ainsi que la chimerisation / réparation *in vivo* (Volkov A. et al.. (1999) Nucleic Acids Research 27 :e18) sont concernées par l'invention.

6) Le procédé de l'invention peut être utilisé pour quantifier ou comparer la fidélité d'une (de) méthode(s) de polymérisation d'acides nucléiques. L'expérimentateur réalise une/des banque(s) de gènes modèles à partir des produits issus de polymérisation(s) réalisée(s) dans des conditions expérimentales données, composition en tampon, type de polymerase etc...

Dans un deuxième temps il détermine, par la méthode de l'invention, les charges mutationnelles associées aux banques de gène modèle. Ces dernières, après la prise en compte éventuelle du nombre de cycles de réplication, permettent d'estimer la fidélité de la méthode de polymérisation, dans les conditions expérimentales utilisées.

La présente invention concerne également les kits pour la mise en oeuvre du procédé et de ses différentes applications ou utilisations.

Un tel kit comprend :

- au moins un gène modèle par exemple sous la forme d'un plasmide ;
- au moins un abaque permettant de lier la charge mutationnelle d'une banque de gènes obtenue par mutagenèse aléatoire du gène modèle à la fraction de gènes modèles mutés observés dans ladite banque ;
- les réactifs nécessaires à la mise en oeuvre de la technique de mutagenèse aléatoire comme par exemple une ADN polymérase thermostable, dNTP, magnésium, manganèse, tampon de PCR dans le cas où la technique de mutagenèse correspond à la PCR error-prone ;
- les réactifs, si nécessaires, pour observer des gènes modèles mutants ;
- une notice indiquant les sous-facteurs de correction définis pour un paramètre sélectionné comme influençant la charge mutationnelle.

Il y a autant d'abaques que de gènes modèles dans ledit kit.

Dans une forme de mise en oeuvre où la technique de mutagenèse utilise une étape d'amplification, le kit peut posséder les amorces pour l'amplification du gène modèle.

Lorsque le kit est mis en oeuvre pour obtenir et contrôler une charge mutationnelle désirée dans une banque de gènes mutés, il contient en outre au moins un abaque lui permettant de lier la charge mutationnelle (CM) d'une banque de gènes obtenue par mutagenèses aléatoire d'un gène modèle aux conditions expérimentales de mutagenèses aléatoire.

Les sous-facteurs de correction fournis seront complétés par une ou plusieurs des données suivantes, et de façon non limitative, telles que :

- la longueur du gène d'intérêt,
- la composition en base A du gène d'intérêt,

- la composition en base C du gène d'intérêt,
- la composition en base G du gène d'intérêt,
- la composition en base T du gène d'intérêt,
- le nombre de multiplication de la matrice du gène modèle
- le nombre de multiplication de la matrice du gène d'intérêt,

dont les valeurs correspondantes sont intégrées par l'expérimentateur, en fonction du gène d'intérêt. Le choix de ces données se fera selon que l'on souhaite tenir compte d'un ou plusieurs paramètres intrinsèques et/ou expérimentaux.

Dans le cas où le gène modèle correspond à la *gfp* et la technique de mutagenèse correspond à la PCR error-prone, les différents sous-facteurs de correction sont ceux indiqués précédemment.

Dans une forme préférée, le kit possède plusieurs gènes modèles et plusieurs abaques pour chaque gène modèle de manière à choisir le gène modèle qui ait les propriétés intrinsèques les plus proches du gène d'intérêt.

Selon une forme particulière de mise en oeuvre du kit de l'invention, les sous-facteurs de correction sont enregistrés dans un fichier numérique sauvegardé sur un support approprié (CD-Rom, disquette, etc.).

De même, dans un kit selon l'invention, l'abaque peut être enregistré sous forme d'une base de données numériques sauvegardées sur un support approprié.

L'invention se rapporte enfin à un procédé dans lequel l'une au moins des étapes (a), (c) et (d) est mise en oeuvre par des moyens informatiques.

Lesdits moyens informatiques peuvent :

- lier la charge mutationnelle d'une banque de gènes obtenue par mutagenèse aléatoire d'un gène modèle à la fraction de gènes modèles mutés observés dans ladite banque.
- être incrémentés par chaque nouvel abaque créé. Ce procédé intègre la mise en oeuvre d'une base de données de différents abaques, chacun défini pour une technique de mutagenèse et un gène modèle donné.
- posséder les données pour établir au moins un facteur de correction pour la détermination de la charge mutationnelle d'une banque de gènes d'intérêt mutés.

L'expérimentateur devra alors préciser, en fonction de ses travaux, des données numériques relatives à l'une au moins des caractéristiques suivantes, que lesdits moyens informatiques exploitent:

- le gène modèle et ses propriétés intrinsèques
- la technique de mutagenèse
- le ou les paramètres sélectionnés comme capables d'influencer la charge mutationnelle d'une banque de gènes par rapport à une autre pour définir les sous-facteurs de correction.

Lesdits moyens informatiques peuvent être présentés sous la forme d'un logiciel.

Ledit logiciel peut être utilisé sur une calculette préprogrammée, ou un micro-ordinateur, et comporte une interface simple permettant à l'expérimentateur de préciser les différentes données numériques exploitées par lesdits moyens informatiques.

Ledit logiciel permet aussi d'intégrer les données liées aux abaques du ou des gènes modèles, et aux sous-facteurs de correction.

Lesdits abaques et sous-facteurs de correction peuvent être soient directement enregistrés par l'expérimentateur dans ledit logiciel, soit être enregistrés dans un fichier numérique sauvegardé sur un support approprié (CD-Rom, disquette, etc.), ledit support étant introduit dans le micro-ordinateur ou la calculette pré-programmée (ordinateur, calculette, etc.) par l'expérimentateur au moment souhaité.

A titre d'exemple, l'expérimentateur entrera une ou plusieurs des données déjà indiquées précédemment.

Ainsi, l'invention se rapporte aussi :

- d'une part à une calculette préprogrammée ou un micro-ordinateur pour la mise du procédé ci-dessus, et
- d'autre part à un dispositif susceptible d'être mis en oeuvre dans un procédé pour déterminer la charge mutationnelle d'une banque obtenue à l'issue de la mutagenèse aléatoire d'un gène d'intérêt comprenant un kit défini précédemment et ce micro-ordinateur ou cette calculette pré-programmée.

D'autres avantages et caractéristiques de l'invention apparaîtront dans les exemples qui suivent, où il sera fait référence aux dessins en annexe dans lesquels :

La figure 1 représente la séquence du gène de la *gfp*.
La figure 2 représente la relation entre la fraction de clones exprimant une GFP fonctionelle et la charge mutationnelle dans la banque de gènes *gfp* mutés.

La figure 3 représente la matrice des mutations Mut$_{GFP}$ pour le gène modèle *gfp* et pour la technique de mutagènese aléatoire par PCR error-prone.

La figure 4 représente la matrice cible de mutation M$_{target}$[*gfp*] sur le gène *gfp* pour la technique de mutagènèse aléatoire par PCR error-prone.

La figure 5 représente la plasticité de TEM-1 β-lactamase, soit le taux de survie des protéines mutées en fonction de la charge mutationnelle des banques de gènes *TEM-1* mutés.

La figure 6 représente l'évolution de TEM-1 β-lactamase, soit la fréquence d'apparition de clones résistant à la cefotaxime en fonction de la charge mutationnelle des banques de gènes *TEM-1* mutés.

Exemple 1 : Détermination de la charge mutationnelle introduite dans une banque de gènes de xylanase de *Thermotoga maritima* (*xynA*) à l'issue d'une étape de mutagènèse aléatoire grâce à la mise en oeuvre des étapes suivantes :

1) Sélection du gène modèle

Dans l'exemple développé, le gène de la Green Fluorescent Protein (*gfp*) a été choisi comme gène modèle grâce à la facile mise en oeuvre d'un test phénotypique basé sur l'expression du produit du gène *gfp* : les colonies exprimant une protéine correspondant à la « Green Fluorescent Protein » ou GFP fonctionnelle présentent une couleur verte aux ultraviolets.

2) Sélection de la méthode de mutagènèse aléatoire

Du fait de leur facilité de mise en oeuvre, les mutagenèses aléatoires ont été menées par PCR error-prone comme décrit par Cadwell (Cadwell R.C., Joyce G.F. (1992) PCR Methods Appl. 2 :28-33).

3) Obtention d'un abaque permettant de lier la charge mutationnelle d'une banque de gènes modèle *gfp* mutés aléatoirement à la fraction de gènes modèle mutés observés dans ladite banque

Pour déterminer la correspondance entre la charge mutationnelle dans la banque de gènes modèle *gfp* mutés et la fraction de clones exprimant une GFP fonctionnelle, trois expériences de mutagènese aléatoire (A,B et C) par PCR error-prone ont été effectuées.

Les réactions de PCR error-prone ont été réalisées dans des volumes de 100μL contenant 10mM de Tris-HCl pH9, 50mM de KCl, 0.1% de Triton X-100, 0.2mg/mL de BSA, 20pmol d'amorces universelles pET5' et pET3', 2,5U de T*aq* polymerase (QBiogen). Les concentrations en cations divalents sont de 4mM MgCl$_2$ pour l'expérience A, 7mM MgCl$_2$ plus 0,3mM MnCl$_2$ pour l'expérience B et 7mM de MgCl$_2$ plus 0,5mM MnCl$_2$ pour l'expérience C. Les concentrations en dNTP utilisées sont 0,2mM de dATP, 0,2mM de dGTP, 1mM de dTTP et 1mM de dCTP tandis que 1fmol de vecteur a servi comme matrice ADN. Un programme PCR de 94°C pendant 5min; (91°C pendant 30s, 60°C pendant 30s, 72°C pendant 1min.) 25 fois puis 72°C pendant 10 min en guise d'étape d'élongation finale, a été appliqué dans un thermocycler MJ Research PTC-200.

Les produits PCR ont alors été purifiés en utilisant le kit de purification sur colonne QIAquick (Qiagen), vérifiés sur gel et les quantités d'ADN obtenues ont été quantifiées par le kit PicoGreen (Molecular Probes) comme décrit par le fournisseur.

Les produits de PCR error-prone (1μg) ont été digérés par Eco *RI* et Nde *I* (New England Biolabs) pendant 1h à 37°C dans le tampon de réaction adéquat comme décrit par le fournisseur puis purifiés sur colonne QIAquick et insérés dans le vecteur pET26b+ (Stratagene) préalablement digéré avec Eco *RI* et Nde *I.* Les cellules *E. coli* MC1061(DE3) ont été transformées par choc thermique comme décrit par Maniatis (Sambrook J. et al. (1989) Molecular cloning: a laboratory manual Cold Spring Harbor Laboratory Press - New York) avec 1μL des réactions de ligation. Les transformants ont été sélectionnés sur boites LB agar complémentées avec 0,6mg/mL de kanamycine.

Après clonage et transformation, les trois banques sont analysées pour la fraction de clones exprimant une GFP fonctionnelle mais également par séquençage pour déterminer les charges mutationnelles dans les trois banques de gènes *gfp* mutés à l'issue des expériences de mutagènèse A, B et C.

Le criblage des clones exprimant une protéine GFP fonctionnelle a été réalisé en identifiant les colonies présentant une fluorescence verte sous banc UV (longueur d'onde d'excitation à 355nm). Parallèlement, le rendement d'insertion des gènes *gfp* mutés dans les différentes banques a été analysé par PCR sur des colonies choisies aléatoirement. Par banque, le criblage pour la recherche de clones présentant une GFP fonctionnelle, a été réalisé sur environ 1000 clones et le rendement d'insertion (nombre de clones présentant un insert par nombre total de clones) a été estimé à partir de 96 PCR sur colonies, ceci de façon à être statistiquement pertinent.

Les banques de gènes *gfp* mutés sont ainsi analysées sur le nombre total de colonie (cfu_total), le nombre de colonies exprimant une GFP fonctionnelle (cfu_fluo) et le rendement d'insertion des gènes *gfp* mutés dans les banques (Ri). La fraction de clones exprimant une GFP fonctionnelle dans une banque donnée (Fa[GFP]) est alors déduite de l'équation suivante:

$$Fa[GFP] = \frac{cfu\_fluo}{cfu\_total \times Ri}$$

Pour déterminer les charges mutationnelles dans les banques A, B ou C de gènes *gfp* mutés, 24 clones par banque contenant un insert *gfp* ont été sélectionnés pour une amplification PCR avec les amorces pET5'/pET3'. Les produits de digestion ont été purifiés sur colonne QIAquick puis séquencés en totalité. Toutes les mutations (modification de base, insertion et délétion) détectées dans les séquences par rapport à celle présentée par le gène *gfp* sauvage ont été vérifiées sur les chromatogrammes.

Pour chacune des trois banques, le séquençage a porté sur 13623 à 15774 pb. Le séquençage d'un nombre suffisamment important de bases permet d'obtenir une mesure statistiquement fiable des charges mutationnelles, même dans le cas de la banque contenant les gènes les moins mutés (banque A). Il est à noter que le ratio entre les clones exprimant une GFP fonctionnelle et ceux exprimant une GFP inactive a été conservé pour la sélection des 24 clones dédiés au séquençage. En effet, il a été décrit qu'à l'issue d'une étape de mutagènese aléatoire les gènes codant pour une protéine active pourraient avoir une charge mutationnelle plus faible que des gènes non sélectionnés (Daugherty P et al. (2000) Proc. Natl. Acad. Sci. USA. 97 :2029-2034). Ainsi, la sélection aléatoire pour le séquençage d'un nombre statistiquement faible de clones aurait pu biaiser l'observation de la charge mutationnelle.

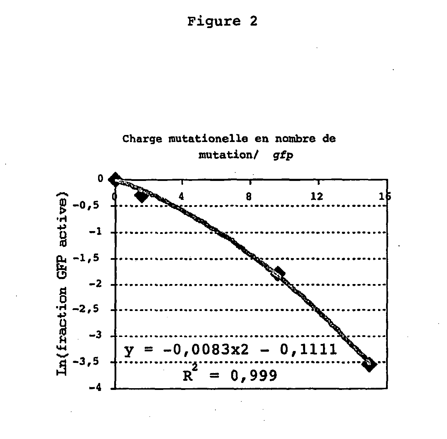

Le tableau 2 ci-après représente l'analyse de la fraction de clone exprimant une GFP fonctionnelle en fonction de la charge mutationnelle des banques de *gfp* mutés. Il résume les résultats obtenus sur l'analyse du test phénotypique GFP, tandis que la figure 2 présente le logarithme de la fraction de GFP fonctionnelle en fonction de la charge mutationnelle dans les banques de *gfp* mutés.

Tableau 2

| Expérience PCR-ep | Conditions réactionelles | | Nombre de pb séquencées | Nombre de mutations | Charge mutationelle (mutatlons/gfp) | Fraction GFP fonctionnelle (%) |
|---|---|---|---|---|---|---|
| | [MgCl2] mM | [MnCl2] | | | | |
| A | 4 | 0 | 15057 | 33 | 1,57 | 75,1 |
| B | 7 | 0.3 | 15774 | 212 | 9,64 | 16,5 |
| C | 7 | 0.5 | 13623 | 285 | 15 | 2,9 |

A partir des données générées, il est possible d'établir une courbe de régression polynomiale d'ordre 2, représentant l'abaque selon l'équation suivante :

$$\ln(Fa[GFP]) = -0,0083 \times CM[gfp]^2 - 0,1111 \times CM[gfp]$$

liant la charge mutationnelle d'une banque de gènes obtenue par PCR error-prone du gène modèle *gfp* (C.M.[*gfp*]) à la fraction de gènes *gfp* mutés observés dans ladite banque lors du test phénotypique (Fa[GFP]).

La valeur du coefficient de régression ($R^2$) étant de 0,999 (cf figure 2), l'abaque précédemment déterminé permet d'estimer de façon précise la charge mutationnelle dans une banque de gènes *gfp* mutés à l'issue d'une étape de mutagènese aléatoire par PCR error-prone à partir de la fraction de clone exprimant une GFP fonctionnelle.

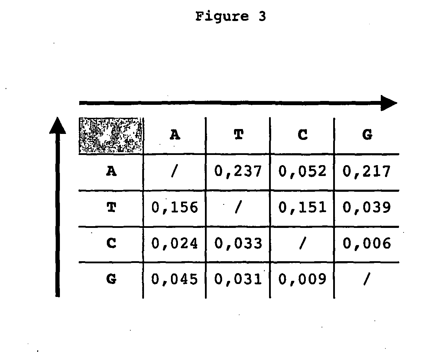

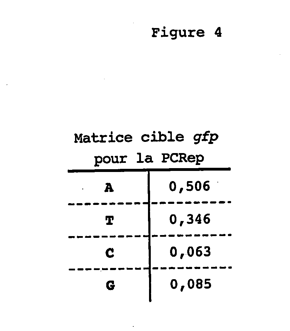

L'analyse des séquençages sur les gènes *gfp* mutés a permis également d'analyser la matrice de mutation $Mut_{GFP}$ (figure 3) et d'en déduire la matrice cible de mutation sur le gène *gfp*, $M_{target}[gfp]$ (figure 4).

4) Application en parallèle de la mutagènèse aléatoire au gène modèle *gfp* et au gène d'intérêt *xynA* de *Thermotoga maritima*

Le gène *xynA* de xylanase issue de *Thermotoga maritima* (numéro d'accession EMBL Z46264) a été soumis à mutagenèse aléatoire par PCR error-prone avec 7mM $MgCl_2$ et 0,3mM $MnCl_2$ (conditions B). Le protocole expérimental est identique à celui précédemment décrit, excepté que l'étape d'élongation PCR a été fixée à 3 minutes au lieu de 1 min. Il est à noter que le même mélange réactionnel a servi pour la PCR error-prone sur le gène *gfp* et pour la PCR error-prone sur le gène *xynA*. L'utilisation du gène *gfp* comme standard interne dans le cadre de l'invention doit permettre de s'affranchir des variations expérimentales sur les charges mutationnelles dans les banques de gènes modèle *gfp* mutés.

Les rendements des expériences de PCR error-prone ont été déterminés par comparaison avec une quantité connue de standard ADN après migration de 1μL de réaction sur gel agarose 1%. La quantité initiale de vecteur étant de 1fmole dans chacune des deux expériences menées, le nombre de multiplication (d) a pu être déterminé à 15 et 12 pour les PCR error-prone *gfp* et *xynA* respectivement.

5) Détermination de la charge mutationnelle de la banque obtenue à partir du gène modèle *gfp*

A l'issue de la PCR error-prone réalisée dans les conditions B, la fraction de gènes *gfp* codant pour une protéine fonctionnelle dans la banque a été déterminée à 0.165.

En utilisant l'abaque donné par l'équation suivante (équation 4) :

$$\ln(\mathrm{Fa[GFP]}) = -0{,}0083 \times \mathrm{CM}[gfp]^2 - 0{,}1111 \times \mathrm{CM}[gfp]$$

liant la charge mutationnelle d'une banque de gènes modèle *gfp* mutés à la fraction de gènes modèle mutés observés, la charge mutationnelle dans la banque de *gfp* mutés est simulée à 9,5 mutations par gène.

Il est à noter que la charge mutationnelle dans cette même banque a été déterminée par séquençage à 9,64 mutations par gène *gfp* soit une erreur de 1,5% entre la charge mutationnelle donnée par l'abaque et celle déterminée par séquençage.

6) Détermination de la charge mutationnelle introduite dans la banque de gènes *xynA* mutés obtenue à partir de la charge mutationnelle dans la banque de gènes modèle *gfp* mutés

La charge mutationnelle introduite dans la banque de gènes *xynA* mutés à l'issue de l'expérience de PCR error-prone est déterminée en corrigeant la charge mutationnelle calculée dans la banque de gènes modèle *gfp* mutés avec le facteur de correction C.F. définie selon l'équation suivante :

$$\mathrm{C.M.\ (gène\ d'intérêt) = C.M.\ (gène\ modèle)\ x\ C.F.}$$

dans laquelle, C.F. représente le facteur de correction, et C.M. représente la charge mutationnelle.

a) En tenant compte d'au moins un paramètre intrinsèque

Dans cette analyse, le paramètre intrinsèque retenu est la longueur des gènes, ce dernier étant reconnu par l'homme de métier comme ayant une forte influence sur la charge mutationnelle des banques de gènes mutés.

Le gène modèle correspondant à la *gfp* et la technique de mutagenèse correspondant à la PCR error-prone, l'unique sous-facteur de correction correspond pour :

paramètre intrinsèque 1 = longueur des gènes
Avec C.F.[L] = L(gène d'intérêt)/717 où L est en nombre de paire de base.

Le tableau 3 ci-après reprend les principaux paramètres intrinsèques, notamment la longueur du gène modèle *gfp* et du gène d'intérêt *xynA*.

Tableau 3

| Gène | L (pb) | Composition en base (%) | | | |
|------|--------|---|---|---|---|
| | | A | T | C | G |
| *gfp* | 717 | 34 | 26 | 19,7 | 20,3 |
| *xynA* | 3120 | 32 | 19 | 23 | 26 |

Ainsi la résolution donne :

$$\text{C.F.} = \text{C.F.[L]} = 3120/717 = 4,35$$

A l'issue de la simulation avec la longueur comme unique paramètre intrinsèque, la charge mutationnelle calculée dans la banque de gène *xynA* est de :

$$\text{C.M.[xynA]}siml = 4,35 \times 9,5 = 41,3 \text{ mutations/gène } xynA$$

b) <u>En tenant compte d'au moins deux paramètres intrinsèques et d'un paramètre expérimental</u>

Dans cette analyse, plus poussée que la précédente, les paramètres intrinsèques retenus sont la longueur des gènes et la composition en base. Le paramètre expérimental retenu est le nombre de multiplication de la matrice ADN lors de la PCR error-prone. Ces paramètres sont connus de l'homme de métier comme pouvant influencer la charge mutationnelle dans les banques de gènes à l'issue d'une mutagenèse par PCR error-prone.

Le gène modèle correspondant à la *gfp* et la technique de mutagenèse correspondant à la PCR error-prone, différents sous-facteurs de correction correspondent pour :

- paramètre intrinsèque 1 représente la longueur des gènes selon l'équation suivante :

$$\text{C.F.[L]} = \text{L(gène d'intérêt)}/717$$

où L est en nombre de paire de base.

- paramètre intrinsèque 2 représente la composition en base sur les brins codant d'ADN selon l'équation suivante :

$$\text{Avec C.F.[base]} = \left( \frac{0{,}506 \times A_{g.intér\hat{e}t}}{0{,}34} + \frac{0{,}346 \times T_{g.intér\hat{e}t}}{0{,}26} + \frac{0{,}063 \times C_{g.intér\hat{e}t}}{0{,}197} + \frac{0{,}085 \times G_{g.intér\hat{e}t}}{0{,}203} \right)$$

où $A_{g.interêt}$, $T_{g.interêt}$, $C_{g.interêt}$ et $G_{g.interêt}$ sont en fractions.

- paramètre expérimental représente le taux de multiplication selon l'équation suivante :

$$\text{C.F.[d]} = \text{d(gène d'interêt)} / \text{d(gfp)}$$

Le tableau 3 reprend les principaux paramètres intrinsèques, longueur et composition en base, du gène modèle *gfp* et du gène d'intérêt *xynA*. Par ailleurs, à l'issue des PCR error-prone réalisées sur le gène modèle *gfp* et le gène d'intérêt *xynA,* les taux de multiplication sont de 15 et 12 respectivement pour *gfp* et *xynA* .

La résolution donne :

$$\text{C.F.[L]} = 3120/717 = 4,35$$

$$\text{C.F.[d]} = 12/15 = 0,80$$

$$C.F.[base] = \left( \frac{0,506 \times 0,32}{0,34} + \frac{0,346 \times 0,19}{0,26} + \frac{0,063 \times 0,23}{0,197} + \frac{0,085 \times 0,26}{0,203} \right) = 0,91$$

La construction du facteur de correction C.F. s'effectuant par la multiplication des différents sous-facteurs pris en compte pour la détermination de la charge mutationnelle de la banque de gènes d'intérêt mutés :

```
C.F. = C.F.[L]× C.F.[base]× C.F.[d]
```

Soit :

```
C.F. = 4,35×0,91×0,8=3,17
```

A l'issue de la simulation avec la longueur et la composition en base comme paramètres intrinsèques et le taux de multiplication comme paramètre expérimental, la charge mutationnelle calculée dans la banque de gènes *xynA* mutés est de :

```
C.M.[xynA]sim2  =  3,17  ×  9,5  =  30,1
mutations/gène xynA
```

La charge mutationnelle dans la banque de gènes *xynA* mutés a également été déterminée par séquençage pour valider les méthodes de détermination de charge mutationnelle découlant de l'invention.

Douze clones recombinant *xynA Thermotoga maritima* mutés ont été sélectionnés pour séquençage. Les gènes xylanases mutés ont été entièrement séquencés comme décrit dans l'exemple 1, (3).

Parmi les 38160pb séquencées, 381 mutations ont été détectées dans les gènes mutés *xynA.* Ainsi la charge mutationnelle observée dans la banque de gènes *xynA* mutés est de 31,8 mutations / gène *xynA.*

L'erreur constatée entre la charge mutationnelle observée et la charge mutationnelle simulée dans la banque de gène *xynA* est de 29,9% pour la simulation 1 (réalisée avec la seule longueur comme paramètre intrinsèque), et de 5,3% pour la simulation 2 (réalisée avec la longueur et la composition en base comme paramètres intrinsèques et le taux de multiplication comme paramètre expérimental).

La prise en compte, dans les simulations, de la composition en base et du taux de multiplication du gène modèle *gfp* et du gène d'intérêt *xynA* permet de diminuer l'erreur sur la charge mutationnelle d'environ un facteur six. Un tel résultat souligne l'intérêt, présenté par l'invention, d'intégrer dans le facteur de correction C.F. non pas la seule longueur des gènes mais le plus grand nombre de paramètres intrinsèques et expérimentaux identifiés comme susceptibles d'influencer la charge mutationnelle.

Par ailleurs la justesse du résultat de la charge mutationnelle obtenu dans le cadre de la simulation 2 par rapport à celle observée lors d'un séquençage (moins de 6% d'erreur) démontre la pertinence de l'invention à pouvoir remplacer de manière fiable l'étude de la charge mutationnelle dans une banque de gènes mutés par séquençage.

L'ensemble des résultats sur les charges mutationnelles simulées ou observées dans la banque de gènes *xynA* mutés est présenté dans le tableau 4 ci-après.

Ce tableau 4 récapitule les résultats de charge mutationnelle sur une banque de gènes *xynA* mutés, observée par séquençage (C.M.[xynA]obs), simulée en tenant compte uniquement de la longueur des gènes (simulation A) ou simulée en tenant compte de la longueur des gènes, de la composition en base et du nombre de multiplication de la matrice ADN (simulation B).

Tableau 4

| Simulation | C.M.[xynA]*sim* | Erreur sim./obs. |
|---|---|---|
| A | 41,3 | 29,9% |
| B | 30,1 | 5,3% |

(suite)

| Simulation | C.M.[xynA]*sim* | Erreur sim./obs. |
|---|---|---|
| C.M.[xynA]*obs* | 31,8 | |

Exemple 2. Analyse de la plasticité protéique de TEM-1 β-lactamase

Cette analyse est effectuée selon les étapes suivantes :

1) sélection du gène modèle ;
2) sélection de la méthode de mutagènese aléatoire ;
3) obtention d'un abaque permettant de lier la charge mutationnelle d'une banque de gènes modèle *gfp* mutés aléatoirement à la fraction de gènes modèle mutés observés dans ladite banque ;

Ces étapes sont identiques à celles développées dans l'exemple 1. Par contre cet exemple comprend les étapes 4 à 7 décrites ci-après.

4) Application en parallèle de la mutagenèse aléatoire par PCR error-prone au gène modèle *gfp* et au gène d'intérêt *TEM-1*

Le gène modèle *gfp* et le gène d'intérêt *TEM-1* (numéro d'accession EMBL AAR25033) ont été soumis à quatres expériences de mutagenèse aléatoire par PCR error-prone avec 4mM MgCl$_2$ (condition I), 7mM MgCl$_2$ plus 0,15mM MnCl$_2$ (condition II), 7mM MgCl$_2$ plus 0,3mM MnCl$_2$ (condition III) et 7mM MgCl$_2$ plus 0,5mM MnCl$_2$ (condition IV). Le protocole expérimental est identique à celui décrit dans l'exemple 1.

Les rendements des expériences de PCR error-prone ont été déterminés comme décrit dans l'exemple 1. Le nombre de multiplication a ainsi pu être déterminé à 15 et 14 pour les PCR error-prone *gfp* et *TEM-1* respectivement.

5) Détermination des charges mutationnelles dans les banques obtenues à partir du gène modèle *gfp*

A l'issue des quatre expériences de PCR error-prone, les fractions des banques de gènes *gfp* mutés codant pour une protéine GFP fonctionnelle ont été déterminées avec l'équation suivante (équation 3) :

$$Fa[GFP] \approx \frac{cfu\_fluo}{cfu\_total \times Ri}$$

La détermination des charges mutationnelles dans les banques de gènes *gfp* mutés est effectuée en utilisant l'abaque décrit dans l'équation suivante (équation 4) :

$$\ln(Fa[GFP]) = -0,0083 \times CM[gfp]^2 - 0,1111 \times CM[gfp]$$

à partir de la fraction de gène *gfp* codant pour une protéine GFP active (Fa[GFP]).

Le tableau 5 ci-après reprend les résultats obtenus à l'issue de cette analyse. Il donne les charges mutationnelles dans les banques de gènes *gfp* mutés à partir de l'analyse du test phénotypique mis en place sur la protéine GFP. Ri est le rendement d'insertion des gènes *gfp* mutés dans les différentes banques, déterminé par PCR/colonie; cfu_totales et cfu_fluo sont respectivement le nombre de clones analysés et le nombre de clones fluorescents verts aux U.V.; Fa[GFP] est la fraction fonctionnelle de clones GFP tandis que C.M.[gfp] est la charge mutationnelle dans les différentes banques de gènes *gfp* mutés.

**Tableau 5**

| Conditions PCR-ep | cfu_totales | cfu_fluo | Ri | Fa[GFP] | **C.M.[*gfp*]** |
|---|---|---|---|---|---|
| **I** | 270 | 226 | 0,958 | 0,874 | **1,1** |
| **II** | 340 | 179 | 0,958 | 0,550 | **4,1** |

(suite)

| Conditions PCR-ep | cfu_totales | cfu_fluo | Ri | Fa[GFP] | C.M.[*gfp*] |
|---|---|---|---|---|---|
| III | 775 | 135 | 0,958 | 0,182 | **9,1** |
| IV | 1160 | 52 | 0,958 | 0,047 | **13,6** |

6) Détermination des charges mutationnelles introduites dans les banques de gènes *TEM-1* mutés obtenues à partir des charges mutationnelles dans les banques de gènes modèle *gfp* mutés

Comme précédemment, les charges mutationnelles introduites dans les banques de gènes *TEM-1 mutés* à l'issue des expériences de PCR error-prone sont déterminées en corrigeant les charges mutationnelles calculées dans les banques de gènes modèle *gfp* mutés avec le facteur de correction C.F. selon l'équation suivante (équation 1) :

$$\text{C.M. (gène d'intérêt) = C.M. (gène modèle) x C.F.}$$

où C.F. représente le facteur de correction et C.M. représente la charge mutationnelle.

Pour obtenir un maximum de précision sur la détermination des charges mutationnelles dans les banques de gènes *TEM-1* mutés, le facteur de correction est calculé en tenant compte de deux paramètres intrinsèques (la longueur et la composition en base des gènes) et d'un paramètre expérimental (le taux de multiplication des matrices ADN lors de la PCR error-prone).

Le gène modèle correspondant à la *gfp* et la technique de mutagenèse correspondant à la PCR error-prone, différents sous-facteurs de correction correspondent pour :

- paramètre intrinsèque 1 représente la longueur des gènes selon l'équation suivante :

$$\text{C.F.[L] = L(gène d'intérêt)/717}$$

où L est en nombre de paire de bases.

- paramètre intrinsèque 2 représente la composition en bases sur les brins codant d'ADN selon l'équation suivante :

$$\text{Avec C.F.[base]} = \left( \frac{0{,}506 \times A_{g.intérêt}}{0{,}34} + \frac{0{,}346 \times T_{g.intérêt}}{0{,}26} + \frac{0{,}063 \times C_{g.intérêt}}{0{,}197} + \frac{0{,}085 \times G_{g.intérêt}}{0{,}203} \right)$$

où $A_{g.intérêt}$, $T_{g.intérêt}$, $C_{g.intérêt}$ et $G_{g.intérêt}$ sont en fraction.

- paramètre expérimental représente le taux de multiplication selon l'équation suivante :

$$\text{C.F.[d] = d(gène d'intérêt) / d(gfp)}$$

Le tableau 6 reprend les principaux paramètres intrinsèques, longueur et composition en bases du gène modèle *gfp* et du gène d'intérêt *TEM-1.* Par ailleurs, à l'issue des PCR error-prone réalisées sur le gène modèle *gfp* et le gène d'intérêt *xynA,* les taux de multiplication sont de 15 et 14 pour *gfp* et *TEM-1* respectivement.

Tableau 6. Paramètres intrinsèques du gène modèle *gfp* et du gène d'intérêt *TEM-1*.

| Gène | L (pb) | Composition en base (%) | | | |
|------|--------|-----|-----|-----|-----|
| | | A | T | C | G |
| *gfp* | 717 | 34 | 26 | 19,7 | 20,3 |
| *TEM-1* | 861 | 25,9 | 24,7 | 23,6 | 25,8 |

La résolution donne :

$$C.F.[L] = 861/717 = 1,2$$

$$C.F.[base] = \left( \frac{0,506 \times 0,259}{0,34} + \frac{0,346 \times 0,247}{0,26} + \frac{0,063 \times 0,236}{0,197} + \frac{0,085 \times 0,258}{0,203} \right) = 0,9$$

$$C.F.[d] = 14/15 = 0,93$$

Soit :

$$C.F. = 1,2 \times 0,9 \times 0,93 = 1,01$$

Dans la détermination du facteur de correction C.F., la plus grande longueur du gène *TEM-1* est compensée par un plus fort pourcentage en base C et G et une diminution du rendement de PCR error-prone dans le cas du gène *TEM-1* par rapport au gène modèle *gfp*.

A l'issue de la simulation avec deux paramètres intrinsèques et un paramètre expérimental, les charges mutationnelles calculées dans les banques de gène *TEM-1* sont de :

- Pour la condition I :

$$C.M.[TEM-1]sim = 1,01 \times 1,1 = 1,1 \text{ mutations/gène } TEM-1$$

- Pour la condition II :

$$C.M.[TEM-1]sim = 1,01 \times 4,1 = 4,1 \text{ mutations/gène } TEM-1$$

- Pour la condition III :

$$C.M.[TEM-1]sim = 1,01 \times 9,1 = 9,2 \text{ mutations/gène } TEM-1$$

- Pour la condition IV :

$$C.M.[\mathit{TEM-1}]sim = 1,01 \times 13,6 = 13,7$$

mutations/gène $\mathit{TEM-1}$

6) <u>Analyse de la plasticité protéique de TEM-1 β-lactamase</u>

L'étude de la plasticité protéique nécessite de pouvoir lier le taux de survie des produits du gène d'intérêt muté, sous un crible donné, dans une banque de gènes d'intérêt mutés en fonction de la charge mutationnelle dans ladite banque (Shafikhani S. et al. (1997) BioTechniques 23 :304-310 / Daugherty P. et al. (2000)Proc. Natl. Acad. Sci. USA 97 :2029-2034).

Pour analyser le taux de survie de TEM-1 β-lactamase dans les quatres banques de PCR error-prone précédemment générées, les produits de PCR (1μg) ont été digérés par Eco *RI* et Nde *I* (New England Biolabs) pendant lh à 37°C dans le tampon de réaction adéquat comme décrit par le fournisseur puis purifiés sur colonne QIAquick et insérés dans le vecteur pET26b+ (Stratagene) préalablement digéré avec Eco *RI* et Nde *I.* Les cellules *E. coli* MC1061(DE3) ont été transformées par choc thermique comme décrit par Maniatis (Sambrook J. et al. (1989) Molecular cloning: a laboratory manual Cold Spring Harbor Laboratory Press -New York) avec 5μL des réactions de ligation. Le nombre de transformants dans les différentes banques a été estimé en étalant une fraction de la banque sur boites LB agar complémentées avec 0,6mg/mL de kanamycine tandis que le nombre de clones exprimant une TEM-1 β-lactamase fonctionnelle a été estimé en étalant le reste de la banque sur boites LB agar complémentées avec 0,6mg/mL de kanamycine et 10μg/mL d'ampicilline. Le rendement d'insertion des gènes mutés *TEM-1* a été estimé par PCR sur colonie comme précédemment décrit.

Le tableau 7 reprend les résultats obtenus sur les taux de survie des protéines de TEM-1 β-lactamase mutées dans les 4 banques d'error-prone. La fraction fonctionnelle de clones TEM-1 (Fa[TEM]), est déterminée à partir du rendement d'insertion des gènes *TEM-1* mutés (Ri), du nombre de clones analysés (cfu_totales) et du nombre de clones résistants sur ampicilline (cfu_R).

Tableau 7 : Taux de survie de la protéine TEM-1 β-lactamase dans les 4 banques de PCR error-prone.

| Conditions | cfu_totales | cfu_R | Ri | **Fa[TEM]** |
|---|---|---|---|---|
| I | 761 | 539 | 0,958 | **0,739** |
| II | 746 | 265 | 0,958 | **0,371** |
| III | 1215 | 55 | 0,938 | **0,048** |
| IV | 1143 | 7 | 0,938 | **0,0065** |

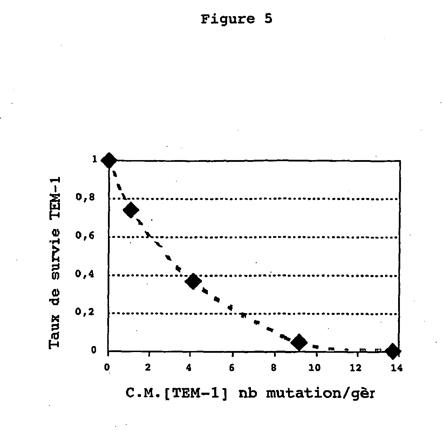

La figure 5 présente l'analyse de la plasticité protéique de TEM-1 Beta-lactamase, à savoir le taux de survie des protéines TEM-1 Beta-lactamase mutées en fonction de la charge mutationnelle dans les banques de gènes *TEM-1* mutés.

En conclusion, la présente invention permet d'étudier, dans une forme particulière de mise en oeuvre, la plasticité protéique sans recours au séquençage.

<u>Exemple 3 : Analyse de l'évolution de TEM-1 Beta-lactamase vers la résistance à l'antibiotique cefotaxime en fonction de la charge mutationnelle des banques de PCR error-prone.</u>

Cette analyse comprend les étapes suivantes:

1) sélection du gène modèle.
2) sélection de la méthode de mutagènese aléatoire.
3) obtention d'un abaque permettant de lier la charge mutationnelle d'une banque de gènes modèle *gfp* mutés aléatoirement à la fraction de gènes modèle mutés observés dans ladite banque.

Ces étapes sont identiques à celles développées dans l'exemple 1 et 2. Par contre, cette analyse comprend aussi les étapes 4-7 décrites ci-après.

4) Application en parallèle de la mutagenèse aléatoire par PCR error-prone au gène modèle *gfp* et au gène d'intérêt *TEM-1*

Le gène modèle *gfp* et le gène d'intérêt *TEM-1* ont été soumis à cinq expériences de mutagenèse aléatoire par PCR error-prone avec 5mM MgCl$_2$ (condition 1), 7mM MgCl$_2$ plus 0,1mM MnCl$_2$ (condition 2), 7mM MgCl$_2$ plus 0,2mM MnCl$_2$ (condition 3), 7mM MgCl$_2$ plus 0,3mM MnCl$_2$ (condition 4) et 7mM MgCl$_2$ plus 0,5mM MnCl$_2$ (condition 5). Le protocole expérimental est identique à celui décrit dans l'exemple 2.
Les rendements des expériences de PCR error-prone ont été déterminés comme décrit dans l'exemple 1 et sont identiques à ceux trouvés dans l'exemple 2. Le taux de multiplication est de 15 et de 14 pour les PCR error-prone *gfp* et *TEM-1* respectivement.

5) Détermination des charges mutationnelles dans les banques obtenues à partir du gène modèle *gfp*

A l'issue des cinq expériences de PCR error-prone, les fractions des banques de gènes *gfp* mutés codant pour une protéine fonctionnelle ont été déterminées avec l'équation suivante (équation 3):

$$Fa[GFP] = \frac{cfu\_fluo}{cfu\_total \times Ri}$$

La détermination des charges mutationnelles dans les banques de gènes *gfp* mutés est effectuée en utilisant l'abaque décrit dans l'équation suivante :

$$\ln(Fa[GFP]) = -0,0083 \times CM[gfp]^2 - 0,1111 \times CM[gfp]$$

à partir de la fraction de gènes *gfp* mutés codant pour une protéine GFP active (Fa[GFP]).
Le tableau 8 ci-après reprend les résultats obtenus à l'issue de cette analyse. Ce tableau rapporte la détermination des charges mutationnelles dans les banques de gènes *gfp* mutés à partir de l'analyse du test phénotypique GFP. Ri est le rendement d'insertion des gènes *gfp* mutés dans les différentes banques, cfu_totales et cfu_fluo sont respectivement le nombre de clones analysés et le nombre de clones fluorescents aux U.V., Fa[GFP] est la fraction fonctionnelle de clones GFP tandis que C.M.[*gfp*] est la charge mutationnelle dans les différentes banques de gènes *gfp* mutés.

Tableau 8

| Conditions PCR-ep | cfu_totales | cfu_fluo | Ri | Fa[GFP] | C.M.[*gfp*] |
|---|---|---|---|---|---|
| 1 | 425 | 305 | 0,96 | 0,749 | 2,2 |
| 2 | 450 | 268 | 0,96 | 0,621 | 3,4 |
| 3 | 568 | 212 | 0,96 | 0,389 | 5,9 |
| 4 | 842 | 100 | 0,96 | 0,124 | 10,5 |
| 6 | 1093 | 26 | 0,75 | 0,032 | 14,8 |

6) Détermination des charges mutationnelles introduites dans les banques de gènes *TEM-1* mutés obtenues à partir des charges mutationnelles dans les banques de gènes modèle *gfp* mutés

Le facteur de correction est calculé en tenant compte de deux paramètres intrinsèques, la longueur et la composition en bases des gènes, et d'un paramètre expérimental, le taux de multiplication des matrices ADN lors de la PCR error-prone.
Le gène modèle *gfp* et le gène d'intérêt *TEM-1* étant identique à celui de l'exemple 2 et les taux de multiplication des matrices *gfp* et *TEM-1* étant également inchangés entre les exemple 1 et 2, le facteur de correction C.F. est identique à celui déterminé dans l'exemple 2.
Soit C.F. = 1,01
A l'issue de la simulation avec deux paramètres intrinsèques et un paramètres expérimental, les charges mutationnelles calculées dans les cinq banques de gènes *TEM-1* mutés sont de :

- Pour la condition 1 :

$$C.M.[TEM-1]sim = 1,01 \times 2,2 = 2,2$$

mutations/gène *TEM-1*

- Pour la condition 2 :

$$C.M.[TEM-1]sim = 1,01 \times 3,4 = 3,4$$

mutations/gène *TEM-1*

- Pour la condition 3 :

$$C.M.[TEM-1]sim = 1,01 \times 5,9 = 6,0$$

mutations/gène *TEM-1*

- Pour la condition 4 :

$$C.M.[TEM-1]sim = 1,01 \times 10,5 = 10,6$$

mutations/gène *TEM-1*

- Pour la condition 5 :

$$C.M.[TEM-1]sim = 1,01 \times 14,8 = 14,9$$

mutations/gène *TEM-1*

7) <u>Analyse de l'acquisition de la résistance à la cefotaxime des clones recombinant TEM-1 β-lactamase mutés</u>

Le système d'évolution choisi décrit l'influence de la charge mutationnelle de la banque de gène *TEM-1* mutés sur la fréquence d'apparition de clones résistant à la cefotaxime grâce à l'évolution du gène *TEM-1.*

Pour analyser la fréquence d'apparition de clones résistants, les produits PCR sont clonés dans le vecteur pET26b+ comme décrit précédemment pour former 5 banques de gènes TEM-1 mutés. Les banques de clones sont réalisées par électrotransformation de cellules *E. coli* MC1061(DE3) avec $10 \times 2\mu L$ de ligation. Le nombre de transformants dans chacune des cinq banques a été estimé en étalant 1/25eme de la banque sur boites LB agar complémentées avec 0,6mg/mL de kanamycine tandis que les clones résistant à la cefotaxime ont été isolés en étalant le reste de la banque (24/25eme) sur boites LB agar complémentées avec 0,6mg/mL de kanamycine et 15ng/mL de cefotaxime. Le rendement d'insertion des gènes *TEM-1* mutés a été estimé par PCR sur colonie.

Le tableau 9 ci-après reprend les résultats obtenus sur la fréquence d'apparition de clones, résistant à la cefotaxime, dans les 5 banques de TEM-mutés. La fréquence de clones améliorés dans les différentes banques est calculée à partir du nombre de clones résistant sur cefotaxime (cfu_cefoR) et du nombre de clones recombinants (Nb_recomb.), lui même déduit du nombre de clones dans la banque (cfu_totales) et du rendement d'insertion des gènes *TEM-1* (Ri) mutés.

Tableau 9

| Conditions | cfu_totales | Ri | Nb_recomb. | cfu_cefbR | **Fréq. améliorés ‰** |
|---|---|---|---|---|---|
| 1 | 103200 | 0,96 | 98900 | 51 | **0,516** |
| 2 | 53750 | 0,96 | 51510 | 41 | **0,796** |

(suite)

| Conditions | cfu_totales | Ri | Nb_recomb. | cfu_cefbR | Fréq. améliorés ‰ |
|---|---|---|---|---|---|
| 3 | 189000 | 0,96 | 181125 | 50 | 0,276 |
| 4 | 158000 | 0,94 | 148125 | 18 | 0,122 |
| 5 | 223500 | 0,92 | 204875 | 11 | 0,054 |

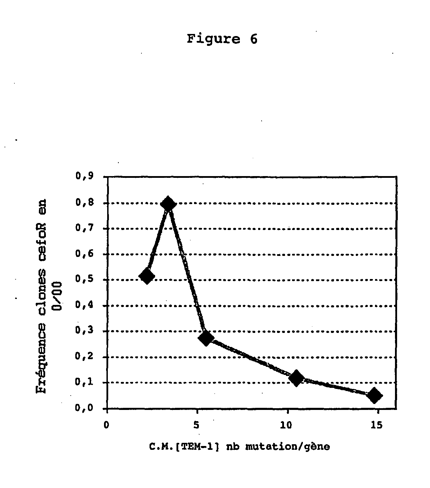

La figure 6 montre l'influence de la charge mutationnelle des banques de gènes *TEM-1* mutés sur la fréquence d'apparition d'un variant TEM-1 amélioré.

La fréquence d'apparition de clones résistants à la cefotaxime en fonction de la charge mutationnelle suit une courbe en cloche passant par une charge mutationnelle optimale.

La méthode de l'invention peut ainsi être utilisée pour analyser les taux d'évolution des banques de gènes mutés mais également pour se placer à la charge mutationnelle optimale et ceci sans recourir au séquençage.

En conclusion, cet exemple montre également l'importance de contrôler précisément la charge mutationnelle dans les banques de gènes mutés puisque la fréquence d'apparition de variants TEM-1 résistants à la céfotaxime est diminuée de trois fois pour une charge mutationnelle passant de 3,4 à 6 mutations par gène *TEM-1.* La présente invention permet, sans séquençage, un contrôle de la charge mutationnelle beaucoup plus fin que les méthodes proposées par l'art antérieur et répond parfaitement à ce type de besoin.

Exemple 4 : comparaison et estimation de la fidélité d'un jeux de ADN polymerases dans une réaction de PCR donnée.

Cette mise en oeuvre particulière de l'invention comprend les étapes suivantes :

1) sélection du gène modèle.
2) sélection de la méthode de mutagènese aléatoire.
3) obtention d'un abaque permettant de lier la charge mutationnelle d'une banque de gènes modèle *gfp* mutés aléatoirement à la fraction de gènes modèle mutés observés dans ladite banque.

Ces étapes sont identiques à celles développées dans les exemple 1 à 3. L'analyse comprend en outre les étapes 4-6 décrites ci-après.

4) Application en parallèle de la PCR au gène modèle *gfp* avec un jeux de ADN polymerases

Le gène modèle *gfp* a été soumis à trois expériences de PCR contenant 7mM de MgCl$_2$ et 0,2mM de MnCl$_2$. Le protocole expérimental est identique à celui décrit dans les exemples précédents avec un temps d'élongation de 1 minute, excepté que 2,5U de T*aq* polymerase (Q-Biogen), de Vent$_R$ (New England Biolabs) ou de Pfu Turbo (Stratagène) ont été utilisée comme ADN polymérase.

Les rendements des expériences de PCR ont été déterminés comme décrit précédemment. Le nombre de multiplication est de $d_A$, $d_B$ et $d_C$ pour les PCR effectuées avec la T*aq* DNA polymerase, la Vent$_R$ DNA polymerase et la Pfu DNA polymerase respectivement.

5) Détermination des charges mutationnelles dans les banques obtenues à partir du gène modèle *gfp*

A l'issue des expériences de PCR, les fractions des banques *gfp* mutés codant pour une GFP fonctionnelle ont été déterminées avec l'équation suivante (équation 3) :

$$Fa[GFP] = \frac{cfu\_fluo}{cfu\_total \times Ri}$$

Les charges mutationnelles dans les banques de gènes *gfp* mutés sont déterminées en utilisant l'abaque décrit dans l'équation suivante (équation 4) :

$$\ln(Fa[GFP]) = -0,0083 \times CM[gfp]^2 - 0,1111 \times CM[gfp]$$

à partir de la fraction de gènes *gfp* mutés codant pour une protéine GFP active (Fa[GFP]).

Les charges mutationnelles sont de A, B et C pour les PCR effectuées avec la Tag DNA polymerase, la Vent$_R$ DNA polymerase et la P*fu* DNA polymerase respectivement.

6) Comparaison de la fidélité des trois polymerases utilisées dans les expériences de PCR

Les taux d'erreur des différentes polymerases dans les conditions réactionnelles utilisées sont déduits des charges mutationnelles dans les banques de gènes *gfp* mutés en normalisant par la longueur du gène modèle *gfp* et les taux de multiplication des PCR.

La longueur du gène modèle *gfp* est de 717 paires de bases et les taux de multiplication à l'issue des PCR sont de d$_A$, d$_B$ et d$_C$ avec la Tag DNA polymerase, la Vent$_R$ DNA polymerase et la Pfu DNA polymerase respectivement.

Ainsi :

$$\text{Taux erreur } [\text{T}aq \text{ DNA polymerase}] = A/(717 \times d_A) \text{ erreur /pb/ duplication}$$

$$\text{Taux erreur } [\text{Vent}_R \text{ DNA polymerase}] = B/(717 \times d_B) \text{ erreur /pb/ duplication}$$

$$\text{Taux erreur } [\text{P}fu \text{ DNA polymerase}] = C/(717 \times d_C) \text{ erreur /pb/ duplication}$$

En conclusion, la méthode de l'invention permet de déterminer précisément les fidélités d'un jeux d'ADN polymerase dans un mélange réactionnel donné sans recourir au séquençage, et ceci en s'affranchissant des variations de conditions expérimentales.

**Revendications**

1. Procédé de détermination de la charge mutationnelle d'une banque de gènes obtenue par mutagenèse aléatoire d'un gène d'intérêt, **caractérisé en ce qu'**il comprend les étapes suivantes :

 a) la préparation d'un abaque liant la charge mutationnelle (CM) d'une banque de gènes obtenue par mutagenèse aléatoire d'un gène modèle à la fraction des gènes modèle mutés observée dans ladite banque ;
 b) la mutagenèse aléatoire du gène modèle ayant servi pour la préparation de l'abaque de l'étape (a) et du gène d'intérêt pour obtenir les banques de gènes mutés correspondantes ;
 c) la détermination de la charge mutationnelle (CM) de la banque de gènes obtenue à partir du gène modèle à l'étape (b) à partir de l'abaque établi à l'étape (a) ;
 d) l'application d'un facteur de correction (CF) à la charge mutationnelle (CM) de la banque de gènes modèle mutés déterminée à l'étape (c) pour déterminer la charge mutationnelle (CM) de la banque de gènes d'intérêt mutés de l'étape (b) ;
 et **en ce que** les étapes (c) et (d) ne comprennent pas de séquençage.

2. Procédé selon la revendication 1,
 **caractérisé en ce que** la technique de mutagenèse mise en oeuvre à l'étape (b) est identique pour le gène modèle et pour le gène d'intérêt et est également similaire à celle de l'étape (a) pour construire l'abaque à partir du gène modèle.

3. Procédé selon l'une quelconque des revendications 1 ou 2, **caractérisé en ce que** pour la préparation de l'abaque de l'étape (a) l'observation des gènes modèle mutés dans ladite banque est réalisée en analysant directement ou indirectement une ou plusieurs modifications d'une ou plusieurs propriétés de la séquence nucléotidique des gènes modèle mutés par rapport au gène modèle avant mutagenèse.

**4.** Procédé selon la revendication 3,
**caractérisé en ce que** ladite observation consiste à observer la modification d'une ou plusieurs propriétés de la protéine codée par le gène modèle.

**5.** Procédé selon la revendication 4,
**caractérisé en ce que** l'observation de la modification d'une ou plusieurs propriétés de la protéine codée par le gène modèle est réalisée par un test phénotypique.

**6.** Procédé selon l'une quelconque des revendications précédentes, **caractérisé en ce que** le gène modèle possède des caractéristiques partagées par un grand nombre de gènes.

**7.** Procédé selon l'une quelconque des revendications précédentes, **caractérisé en ce que** le gène modèle code la protéine GFP.

**8.** Procédé selon l'une quelconque des revendications 1 à 5, **caractérisé en ce que** le gène modèle possède des caractéristiques proches de celles du gène d'intérêt.

**9.** Procédé selon l'une quelconque des revendications 6 ou 8, **caractérisé en ce que** les caractéristiques du gène modèle comprennent :

- sa longueur,
- sa composition en base,
- sa fréquence en bases répétées dans la séquence,
- la présence de séquences symétriques dans le gène.

**10.** Procédé selon l'une quelconque des revendications précédentes, **caractérisé en ce que** la mutagenèse aléatoire des étapes (a) et (b) met en oeuvre la polymérisation.

**11.** Procédé selon la revendication 10,
**caractérisé en ce que** la polymérisation est la PCR error-prone.

**12.** Procédé selon l'une quelconque des revendications précédentes, **caractérisé en ce qu'**à l'étape (b) le même mélange réactionnel est utilisé pour la mutagenèse du gène modèle et du gène d'intérêt.

**13.** Procédé selon l'une quelconque des revendications précédentes, **caractérisé en ce qu'**à l'étape (d), la charge mutationnelle déterminée à l'étape (c) est corrigée en appliquant un facteur de correction (C.F.) qui se décompose en plusieurs sous-facteurs définis suivant les paramètres sélectionnés comme influençant la charge mutationnelle d'une banque de gènes d'intérêt mutés par rapport à une banque de gènes modèle mutés.

**14.** Procédé selon la revendication 13,
**caractérisé en ce que** les paramètres sélectionnés pouvant influencer la charge mutationnelle sont choisis parmi :

- au moins un et de préférence au moins deux paramètres intrinsèques,
- au moins un paramètre expérimental.

**15.** Procédé selon la revendication 14,
**caractérisé en ce que** les paramètres intrinsèques sont choisis parmi les caractéristiques suivantes d'un gène:

- sa longueur,
- sa composition en base
- bases répétées dans la séquence,
- la présence de séquences symétriques dans le gène.

**16.** Procédé selon la revendication 14,
**caractérisé en ce que**, lorsque la mutagenèse est basée sur une amplification en chaîne utilisant une polymérase, les paramètres expérimentaux correspondent à un ou plusieurs des facteurs suivants :

- le taux d'amplification du gène durant l'étape de mutagenèse,
- la quantité de matrice initiale,
- le nombre de cycle d'amplification,
- à la nature et à la quantité de polymérase utilisée
- aux conditions expérimentales utilisées comme la concentration en cations divalents, la concentration et le ratio de concentration des dNTPs.

**17.** Procédé selon l'une quelconque des revendications précédentes, **caractérisé en ce que** au moins un paramètre intrinsèque et au moins un paramètre expérimental sont considérés pour établir le facteur de correction.

**18.** Procédé selon l'une quelconque des revendications précédentes, **caractérisé en ce que** lorsque la mutagenèse est réalisée par PCR error-prone, et le gène modèle est la *gfp,* les différents sous-facteurs de correction sont choisis parmi :

- le paramètre intrinsèque 1 représentant la longueur des gènes selon l'équation suivante :

$$\texttt{C.F.[L] = L(gène d'intérêt)/717}$$

où L est en nombre de paire de base,

- le paramètre intrinsèque 2 représentant la composition en base selon l'équation suivante :

$$\text{C.F.[base]} = \left( \frac{0,506 \times A_{(g.intérêt)}}{0,34} + \frac{0,346 \times T_{(g.intérêt)}}{0,26} + \frac{0,063 \times C_{(g.intérêt)}}{0,197} + \frac{0,085 \times G_{(g.intérêt)}}{0,203} \right)$$

Où $A_{(g.intérêt)}$, $T_{(g.intérêt)}$, $C_{(g.intérêt)}$ et $G_{(g.intérêt)}$ sont en fraction,
- le paramètre expérimental représente le taux de multiplication selon l'équation suivante :

$$\texttt{C.F.[d] = d(gène d'intérêt) / d(gfp)}$$

où d est le taux de multiplication calculé selon l'équation suivante :

$$\texttt{d = ln(Qf/Qi)/ln2}$$

dans laquelle, Qi et Qf représentent respectivement les quantités molaires de la matrice et des produits à l'issue de la PCR.

**19.** Utilisation d'un procédé selon l'une quelconque des revendications 1 à 18 pour contrôler la charge mutationnelle dans une banque de gènes mutés **caractérisée en ce qu'**il comprend une étape supplémentaire de sélection des conditions expérimentales de mutagenèse aléatoire qui seront utilisées pour mettre en oeuvre l'étape (b).

**20.** Utilisation d'un procédé selon l'une quelconque des revendications 1 à 18 pour étudier la capacité d'une protéine à incorporer des mutations tout en conservant une activité présentée par la protéine sauvage.

**21.** Utilisation d'un procédé selon l'une quelconque des revendications 1 à 18 pour étudier la capacité d'une protéine à acquérir une nouvelle propriété avec une charge mutationnelle donnée.

**22.** Utilisation d'un procédé selon l'une quelconque des revendications 1 à 18 pour déterminer la charge mutationnelle optimale dans des banques de gènes d'intérêt mutés pour générer un lot de gènes parentaux avant la mise en oeuvre de méthodes de recombinaison moléculaire.

**23.** Utilisation d'un procédé selon l'une quelconque des revendications 1 à 18 pour quantifier ou comparer la fidélité d'une (de) méthode(s) de polymérisation d'acides nucléiques.

**24.** Kit susceptible d'être mis en oeuvre dans un procédé pour déterminer la charge mutationnelle d'une banque obtenue à l'issue de la mutagenèse aléatoire d'un gène d'intérêt selon l'une quelconque des revendications 1 à 18 ou dans une utilisation selon les revendications 19 à 23, **caractérisé en ce qu'**il comprend:

- au moins un gène modèle par exemple sous la forme d'un plasmide ;
- au moins un abaque permettant de lier la charge mutationnelle d'une banque de gènes obtenue par mutagenèse aléatoire du gène modèle à la fraction de gènes modèles mutés observés dans ladite banque ;
- les réactifs nécessaires à la mise en oeuvre de la technique de mutagenèse aléatoire comme par exemple une ADN polymérase thermostable, dNTP, magnésium, manganèse, tampon de PCR dans le cas où la technique de mutagenèse correspond à la PCR error-prone ;
- les réactifs, si nécessaires, pour observer des gènes modèle mutants ;
- une notice d'utilisation indiquant les sous-facteurs de correction définis pour un paramètre sélectionné comme influençant la charge mutationnelle.

**25.** Kit susceptible d'être mis en oeuvre dans une utilisation selon la revendication 19 **caractérisé en ce qu'**il comprend en plus des composant du kit selon la revendication 24 au moins un abaque permettant de lier la charge mutationnelle (CM) d'une banque de gènes obtenue par mutagenèses aléatoire d'un gène modèle aux conditions expérimentales de mutagenèses aléatoire.

**26.** Kit selon la revendication 24 ou 25, **caractérisé en ce qu'**il possède les amorces pour l'amplification du gène modèle dans le cas où la technique de mutagenèse est basée sur une technique d'amplification.

**27.** Kit selon l'une quelconque des revendications 24 à 26, **caractérisé en ce que** les sous-facteurs de correction fournis doivent être complétés par une ou plusieurs des données comme celles choisies notamment dans le groupe suivant :

- la longueur du gène d'intérêt,
- la composition en base A du gène d'intérêt,
- la composition en base C du gène d'intérêt,
- la composition en base G du gène d'intérêt,
- la composition en base T du gène d'intérêt,
- le nombre de multiplication de la matrice du gène modèle
- le nombre de multiplication de la matrice du gène d'intérêt,

dont les valeurs correspondantes sont intégrées par l'expérimentateur, en fonction du gène d'intérêt et du résultat de mutagenèse aléatoire.

**28.** Kit selon l'une quelconque des revendications 24 à 27, **caractérisé en ce que** le gène modèle est celui de la *gfp.*

**29.** Kit selon la revendication précédente **caractérisé, en ce que** la mutagenèse est réalisée par PCR error-prone, le gène modèle est celui de la *gfp,* les différents sous-facteurs de correction sont choisis parmi :

- le paramètre intrinsèque 1 représentant la longueur des gènes selon l'équation suivante :

$$\texttt{C.F.[L] = L(gène d'intérêt)/717}$$

où L est en nombre de paire de base,

- le paramètre intrinsèque 2 représentant la composition en base sur les brins codant d'ADN selon l'équation suivante :

$$C.F.[\text{base}] = \left( \frac{0,506 \times A_{(\text{g.intérêt})}}{0,34} + \frac{0,346 \times T_{(\text{g.intérêt})}}{0,26} + \frac{0,063 \times C_{(\text{g.intérêt})}}{0,197} + \frac{0,085 \times G_{(\text{g.intérêt})}}{0,203} \right)$$

Où $A_{(\text{g.intérêt})}$, $T_{(\text{g.intérêt})}$, $C_{(\text{g.intérêt})}$ et $G_{(\text{g.intérêt})}$ sont en fraction,
- le paramètre expérimental représente le taux de multiplication selon l'équation suivante :

```
C.F.[d] = d(gène d'intérêt) / d(gfp)
```

où d est le taux de multiplication calculé selon l'équation suivante :

```
d = ln(Qf/Qi)/ln2
```

dans laquelle, Qi et Qf représentent respectivement les quantités molaires de la matrice initiale et des produits à l'issue de la PCR.

30. Kit selon l'une quelconque des revendications 24 à 29, **caractérisé en ce qu'**il possède plusieurs gènes modèles et plusieurs abaques pour chaque gène modèle de manière à choisir le gène modèle ayant des caractéristiques intrinsèques les plus proches du gène d'intérêt.

31. Kit selon l'une quelconque des revendications 24 à 30 **caractérisé en ce que** les sous-facteurs de correction sont enregistrés dans un fichier numérique sauvegardé.. sur un support approprié (CD-Rom, disquette, etc.).

32. Kit selon l'une quelconque des revendications 24 à 31, **caractérisé en ce que** l'abaque est enregistré sous forme d'une base de données numériques sauvegardées sur un support approprié.

33. Procédé selon l'une quelconque des revendications 1 à 18, **caractérisé en ce que** l'une au moins des étapes (a), (c) et (d) est mise en oeuvre par des moyens informatiques.

34. Procédé selon la revendication 33,
**caractérisé en ce que** lesdits moyens informatiques exploitent des données numériques relatives à l'une au moins des caractéristiques suivantes :

- le gène modèle sélectionné
- la technique de mutagenèse choisie
- le ou les paramètres sélectionnés comme capables d'influencer la charge mutationnelle d'une banque de gènes mutés par rapport à une autre pour définir les sous-facteurs de correction.

35. Procédé selon l'une des revendications 33 ou 34, **caractérisé en ce que** lesdits moyens informatiques exploitent des données numériques relatives à l'une au moins des caractéristiques suivantes :

- la longueur du gène d'intérêt,
- la composition en base A du gène d'intérêt,
- la composition en base C du gène d'intérêt,
- la composition en base G du gène d'intérêt,
- la composition en base T du gène d'intérêt,
- le nombre de multiplication de la matrice du gène modèle
- le nombre de multiplication de la matrice du gène d'intérêt,

dont les valeurs correspondantes sont intégrées par l'expérimentateur, en fonction du gène d'intérêt et du résultat de mutagenèse aléatoire.

36. Procédé selon l'une des revendications 33 à 35, **caractérisé en ce que** lesdits moyens informatiques sont utilisés sur une calculette préprogrammée ou un micro-ordinateur.

37. Procédé selon l'une des revendications 33 à 36, **caractérisé en ce que** lesdits moyens informatiques permettent d'intégrer les données liées aux abaques du ou des gènes modèles, et aux sous-facteurs de correction.

38. Un dispositif susceptible d'être mis en oeuvre dans un procédé pour déterminer la charge mutationnelle d'une banque obtenue à l'issue de la mutagenèse aléatoire d'un gène d'intérêt selon l'une quelconque des revendications 1 à 18 et 33 à 37, ou dans une utilisation selon les revendications 19 à 23, **caractérisé en ce qu'**il comprend un kit selon l'une des revendications 24 à 32 et une calculette préprogrammée ou un micro-ordinateur.

**Claims**

1. Method of determining the mutational load of a gene bank obtained by random mutagenesis of a gene of interest, **characterized in that** it comprises the following stages:

    a) preparation of a nomogram linking the mutational load (ML) of a gene bank obtained by random mutagenesis of a model gene to the fraction of mutated model genes observed in said bank;
    b) random mutagenesis of the model gene that was used for preparation of the nomogram in stage (a) and of the gene of interest to obtain the corresponding mutated gene banks;
    c) determination of the mutational load (ML) of the gene bank obtained from the model gene in stage (b) from the nomogram established in stage (a);
    d) application of a correction factor (CF) to the mutational load (ML) of the mutated model gene bank determined in stage (c) to determine the mutational load (ML) of the bank of mutated genes of interest from stage (b);

    and **in that** stages (c) and (d) do not comprise sequencing.

2. Method according to Claim 1, **characterized in that** the mutagenesis technique employed in stage (b) is identical for the model gene and for the gene of interest and is also similar to that of stage (a) for constructing the nomogram based on the model gene.

3. Method according to either of Claims 1 or 2,
**characterized in that** for preparation of the nomogram in stage (a), observation of the mutated model genes in said bank is carried out by analysing, directly or indirectly, one or more changes of one or more properties of the nucleotide sequence of the mutated model genes relative to the model gene before mutagenesis.

4. Method according to Claim 3, **characterized in that** said observation consists of observing the change in one or more properties of the protein encoded by the model gene.

5. Method according to Claim 4, **characterized in that** observation of the change in one or more properties of the protein encoded by the model gene is carried out by a phenotype test.

6. Method according to any one of the preceding claims, **characterized in that** the model gene possesses characteristics that are shared by a large number of genes.

7. Method according to any one of the preceding claims, **characterized in that** the model gene codes for the GFP protein.

8. Method according to any one of Claims 1 to 5, **characterized in that** the model gene possesses characteristics similar to those of the gene of interest.

9. Method according to either of Claims 6 or 8, **characterized in that** the characteristics of the model gene comprise:

    - its length,
    - its base composition,
    - its frequency of bases repeated in the sequence,
    - the presence of symmetrical sequences in the gene.

10. Method according to any one of the preceding claims, **characterized in that** the random mutagenesis in stages (a) and (b) employs polymerization.

11. Method according to Claim 10, **characterized in that** the polymerization is error-prone PCR.

12. Method according to any one of the preceding claims, **characterized in that** in stage (b) the same reaction mixture is used for the mutagenesis of the model gene and of the gene of interest.

13. Method according to any one of the preceding claims, **characterized in that** in stage (d), the mutational load determined in stage (c) is corrected by applying a correction factor (CF), which breaks down into several sub-factors defined according to the parameters selected as influencing the mutational load of a bank of mutated genes of interest relative to a mutated model gene bank.

14. Method according to Claim 13, **characterized in that** the parameters selected that can influence the mutational load are selected from:

   - at least one and preferably at least two intrinsic parameters,
   - at least one experimental parameter.

15. Method according to Claim 14, **characterized in that** the intrinsic parameters are selected from the following characteristics of a gene:

   - its length,
   - its base composition,
   - bases repeated in the sequence,
   - the presence of symmetrical sequences in the gene.

16. Method according to Claim 14, **characterized in that**, when the mutagenesis is based on a chain amplification using a polymerase, the experimental parameters correspond to one or more of the following factors:

   - the degree of amplification of the gene during the stage of mutagenesis,
   - the initial amount of matrix,
   - the number of amplification cycles,
   - the nature and the amount of polymerase used,
   - the experimental conditions used such as the concentration of divalent cations, the concentration and the concentration ratio of the dNTPs.

17. Method according to any one of the preceding claims, **characterized in that** at least one intrinsic parameter and at least one experimental parameter are taken into account for establishing the correction factor.

18. Method according to any one of the preceding claims, **characterized in that** when mutagenesis is carried out by error-prone PCR, and the model gene is *gfp*, the various correction sub-factors are selected from:

   - intrinsic parameter 1 representing the length of the genes according to the following equation:

$$CF\ [L] = L(gene\ of\ interest)/717$$

   where L is in number of base pairs,

   - intrinsic parameter 2 representing the base composition according to the following equation:

$$CF[base] = \left( \frac{0.506 \times A_{(g.interest)}}{0.34} + \frac{0.346 \times T_{(g.interest)}}{0.26} + \frac{0.063 \times C_{(g.interest)}}{0.197} + \frac{0.085 \times G_{(g.interest)}}{0.203} \right)$$

where $A_{(g.interest)}$, $T_{(g.interest)}$, $C_{(g.interest)}$ and $G_{(g.interest)}$ are fractional,

- the experimental parameter represents the multiplication rate according to the following equation:

$$CF\ [d]\ =\ d(gene\ of\ interest)\ /\ d(gfp)$$

where d is the multiplication rate calculated according to the following equation:

$$d\ =\ ln(Qf/Qi)/ln2$$

in which Qi and Qf represent, respectively, the molar amounts of the matrix and of the products resulting from PCR.

19. Use of a method according to any one of Claims 1 to 18 for monitoring the mutational load in a mutated gene bank, **characterized in that** it comprises an additional stage of selection of the experimental conditions of random mutagenesis that will be used for implementing stage (b).

20. Use of a method according to any one of Claims 1 to 18 for investigating the capacity of a protein for incorporating mutations while preserving an activity exhibited by the wild-type protein.

21. Use of a method according to any one of Claims 1 to 18 for investigating the capacity of a protein for acquiring a new property with a given mutational load.

22. Use of a method according to any one of Claims 1 to 18 for determining the optimum mutational load in banks of mutated genes of interest for generating a batch of parental genes prior to the application of methods of molecular recombination.

23. Use of a method according to any one of Claims 1 to 18 for quantifying or comparing the fidelity of a method or methods of polymerization of nucleic acids.

24. Kit that can be used in a method for determining the mutational load of a bank obtained as a result of random mutagenesis of a gene of interest according to any one of Claims 1 to 18 or in a use according to Claims 19 to 23, **characterized in that** it comprises:

   - at least one model gene for example in the form of a plasmid;
   - at least one nomogram for linking the mutational load of a gene bank obtained by random mutagenesis of the model gene to the fraction of mutated model genes observed in said bank;
   - the reagents necessary for the application of the technique of random mutagenesis, for example a thermostable DNA polymerase, dNTP, magnesium, manganese, PCR buffer in the case when the mutagenesis technique corresponds to error-prone PCR;
   - the reagents, if necessary, for observing mutant model genes;
   - instructions for use, indicating the correction sub-factors defined for a selected parameter as influencing the mutational load.

25. Kit that can be employed in a use according to Claim 19, **characterized in that** it comprises, in addition to the components of the kit according to Claim 24, at least one nomogram for linking the mutational load (ML) of a gene bank obtained by random mutagenesis of a model gene to the experimental conditions of random mutagenesis.

26. Kit according to Claim 24 or 25, **characterized in that** it possesses the primers for amplification of the model gene in the case when the mutagenesis technique is based on an amplification technique.

27. Kit according to any one of Claims 24 to 26, **characterized in that** the correction sub-factors supplied must be supplemented with one or more of the data such as those selected notably from the following group:

- the length of the gene of interest,
- the base A composition of the gene of interest,
- the base C composition of the gene of interest,
- the base G composition of the gene of interest,
- the base T composition of the gene of interest,
- the multiplication number of the matrix of the model gene,
- the multiplication number of the matrix of the gene of interest,

the corresponding values of which are inserted by the experimenter, in relation to the gene of interest and the result of random mutagenesis.

28. Kit according to any one of Claims 24 to 27, **characterized in that** the model gene is that of *gfp.*

29. Kit according to the preceding claim, **characterized in that** the mutagenesis is carried out by error-prone PCR, the model gene is that of *gfp*, the various correction sub-factors are selected from:

- intrinsic parameter 1 representing the length of the genes according to the following equation:

$$CF\ [L]\ =\ L(gene\ of\ interest)/717$$

where L is in number of base pairs,

- intrinsic parameter 2 representing the base composition on the DNA coding strands according to the following equation:

$$CF[base] = \left( \frac{0.506 \times A_{(g.interest)}}{0.34} + \frac{0.346 \times T_{(g.interest)}}{0.26} + \frac{0.063 \times C_{(g.interest)}}{0.197} + \frac{0.085 \times G_{(g.interest)}}{0.203} \right)$$

where $A_{(g.interest)}$, $T_{(g.interest)}$, $C_{(g.interest)}$ and $G_{(g.interest)}$ are fractional,

- the experimental parameter represents the multiplication rate according to the following equation:

$$CF\ [d]\ =\ d(gene\ of\ interest)\ /\ d(gfp)$$

where d is the multiplication rate calculated according to the following equation:

$$d\ =\ ln(Qf/Qi)/ln2$$

in which Qi and Qf represent respectively the molar amounts of the initial matrix and of the products resulting from the PCR.

30. Kit according to any one of Claims 24 to 29, **characterized in that** it possesses several model genes and several nomograms for each model gene for selecting the model gene having intrinsic characteristics that are closest to the gene of interest.

**31.** Kit according to any one of Claims 24 to 30, **characterized in that** the correction sub-factors are recorded in a digital file saved on a suitable medium (CD-Rom, floppy disk, etc.).

**32.** Kit according to any one of Claims 24 to 31, **characterized in that** the nomogram is recorded in the form of a digital database saved on a suitable medium.

**33.** Method according to any one of Claims 1 to 18, **characterized in that** at least one of stages (a), (c) and (d) is applied by computerized means.

**34.** Method according to Claim 33, **characterized in that** said computerized means make use of digital data relating to at least one of the following characteristics:

- the model gene selected
- the chosen mutagenesis technique
- the parameter or parameters selected as capable of influencing the mutational load of a mutated gene bank with respect to another for defining the correction sub-factors.

**35.** Method according to either of Claims 33 or 34, **characterized in that** said computerized means make use of digital data relating to at least one of the following characteristics:

- the length of the gene of interest,
- the base A composition of the gene of interest,
- the base C composition of the gene of interest,
- the base G composition of the gene of interest,
- the base T composition of the gene of interest,
- the multiplication number of the matrix of the model gene,
- the multiplication number of the matrix of the gene of interest, the corresponding values of which are inserted by the experimenter, in relation to the gene of interest and the result of random mutagenesis.

**36.** Method according to one of Claims 33 to 35, **characterized in that** said computerized means are used on a preprogrammed pocket calculator or a microcomputer.

**37.** Method according to one of Claims 33 to 36, **characterized in that** said computerized means make it possible to insert data relating to the nomograms of the model gene or genes, and to the correction sub-factors.

**38.** A device that can be used in a method for determining the mutational load of a bank obtained as a result of random mutagenesis of a gene of interest according to any one of Claims 1 to 18 and 33 to 37, or in a use according to Claims 19 to 23, **characterized in that** it comprises a kit according to one of Claims 24 to 32 and a preprogrammed pocket calculator or a microcomputer.

**Patentansprüche**

**1.** Verfahren zur Bestimmung des Mutationsgrades einer durch zufällige Mutagenese eines interessierenden Gens gewonnenen Genbibliothek, **dadurch gekennzeichnet, dass** es die folgenden Schritte umfasst:

a) Erstellen eines Nomogramms, das den Mutationsgrad (MG) einer durch zufällige Mutagenese eines Modellgens gewonnenen Genbibliothek mit der Fraktion der in der Bibliothek beobachteten mutierten Modellgene verknüpft;
b) zufällige Mutagenese des Modellgens, das der Erstellung des Nomogramms aus Schritt (a) gedient hat, und des interessierenden Gens, um die entsprechenden Bibliotheken mutierter Gene zu gewinnen;
c) Bestimmen des Mutationsgrads (MG) der Genbibliothek, die in Schritt (b) aus dem Modellgen gewonnen wurde, aus dem Nomogramm, das in Schritt (a) erstellt wurde;
d) Anwenden eines Korrekturfaktors (KF) auf den Mutationsgrad (MG) der Bibliothek mutierter Modellgene, der in Schritt (c) bestimmt wurde, um den Mutationsgrad (MG) der Bibliothek mutierter interessierender Gene aus Schritt (b) zu bestimmen,

und dadurch, dass die Schritte (c) und (d) keine Sequenzierungen umfassen.

2. Verfahren nach Anspruch 1, **dadurch gekennzeichnet, dass** die in Schritt (b) implementierte Mutagenesetechnik für das Modellgen und für das interessierende Gen identisch ist und auch derjenigen aus Schritt (a) zur Konstruktion des Nomogramms aus dem Modellgen gleicht.

3. Verfahren nach irgendeinem der Ansprüche 1 oder 2, **dadurch gekennzeichnet, dass** für die Erstellung des Nomogramms aus Schritt (a) die Beobachtung der mutierten Modellgene in der Bibliothek durchgeführt wird, indem eine oder mehrere Modifikationen einer oder mehrerer Eigenschaft(en) der Nukleotidsequenz der mutierten Modellgene im Vergleich mit dem Modellgen vor der Mutagenese direkt oder indirekt analysiert wird (werden).

4. Verfahren nach Anspruch 3, **dadurch gekennzeichnet, dass** die Beobachtung darin besteht, die Modifikation einer oder mehrerer Eigenschaft(en) des Proteins, das von dem Modellgen kodiert wird, zu beobachten.

5. Verfahren nach Anspruch 4, **dadurch gekennzeichnet, dass** die Beobachtung der Modifikation einer oder mehrerer Eigenschaft(en) des Proteins, das von dem Modellgen kodiert wird, mit einem Phänotyptest durchgeführt wird.

6. Verfahren nach irgendeinem der vorhergehenden Ansprüche, **dadurch gekennzeichnet, dass** das Genmodell Merkmale aufweist, die von einer großen Anzahl von Genen geteilt werden.

7. Verfahren nach irgendeinem der vorhergehenden Ansprüche, **dadurch gekennzeichnet, dass** das Modellgen das Protein GFP kodiert.

8. Verfahren nach irgendeinem der Ansprüche 1 bis 5, **dadurch gekennzeichnet, dass** das Modellgen Merkmale aufweist, die denen des interessierenden Gens sehr ähnlich sind.

9. Verfahren nach irgendeinem der Ansprüche 6 oder 8, **dadurch gekennzeichnet, dass** die Merkmale des Modellgens Folgendes umfassen:

    - seine Länge,
    - seine Basenzusammensetzung,
    - seine Häufigkeit an Basenwiederholungen in der Sequenz,
    - die Gegenwart von symmetrischen Sequenzen in dem Gen.

10. Verfahren nach irgendeinem der vorhergehenden Ansprüche, **dadurch gekennzeichnet, dass** bei der zufälligen Mutagenese der Schritte (a) und (b) die Polymerisation implementiert wird.

11. Verfahren nach Anspruch 10, **dadurch gekennzeichnet, dass** die Polymerisation die fehlerauslösende PCR ist.

12. Verfahren nach irgendeinem der vorhergehenden Ansprüche, **dadurch gekennzeichnet, dass** in Schritt (b) das gleiche Reaktionsgemisch für die Mutagenese des Modellgens und des interessierenden Gens verwendet wird.

13. Verfahren nach irgendeinem der vorhergehenden Ansprüche, **dadurch gekennzeichnet, dass** in Schritt (d) der in Schritt (c) bestimmte Mutationsgrad korrigiert wird, indem ein Korrekturfaktor (KF) angewendet wird, der sich in mehrere Unterfaktoren aufgliedert, die gemäß den Parametern definiert werden, die ausgewählt wurden, weil sie den Mutationsgrads einer Bibliothek mutierter interessierender Gene bezogen auf eine Bibliothek mutierter Modellgene beeinflussen.

14. Verfahren nach Anspruch 13, **dadurch gekennzeichnet, dass** die ausgewählten Parameter, die den Mutationsgrad beeinflussen können, ausgewählt wurden aus:

    - mindestens einem und bevorzugt mindestens zwei intrinsischen Parametern,
    - mindestens einem experimentellen Parameter.

15. Verfahren nach Anspruch 14, **dadurch gekennzeichnet, dass** die intrinsischen Parameter aus den folgenden Merkmalen eines Gens ausgewählt sind:

    - seiner Länge,

- seiner Basenzusammensetzung,
- seiner Häufigkeit an Basenwiederholungen in der Sequenz,
- der Gegenwart von symmetrischen Sequenzen in dem Gen.

**16.** Verfahren nach Anspruch 14, **dadurch gekennzeichnet, dass**, wenn die Mutagenese auf einer Kettenamplifikation unter Verwendung einer Polymerase basiert, die experimentellen Parameter einem oder mehreren der folgenden Faktoren entsprechen:

- der Amplifikationsrate des Gens während des Mutageneseschritts,
- der Menge an Ausgangsmatrix,
- der Anzahl an Amplifikationszyklen,
- der Art und der Menge der verwendeten Polymerase,
- den verwendeten Versuchsbedingungen, wie der Konzentration an divalenten Kationen, der dNTP-Konzentration und dem dNTP-Konzentrationsverhältnis.

**17.** Verfahren nach irgendeinem der vorhergehenden Ansprüche, **dadurch gekennzeichnet, dass** mindestens ein intrinsischer Parameter und mindestens ein experimenteller Parameter berücksichtigt werden, um den Korrekturfaktor zu erstellen.

**18.** Verfahren nach irgendeinem der vorhergehenden Ansprüche, **dadurch gekennzeichnet, dass**, wenn die Mutagenese mit fehlerauslösender PCR durchgeführt wird und das Modellgen *gfp* ist, die verschiedenen Korrekturunterfaktoren ausgewählt sind aus:

- dem intrinsischen Parameter 1, der für die Länge der Gene gemäß der folgenden Gleichung steht:

$$KF[L] = L(interessierendes\ Gen)/717$$

worin L als Anzahl der Basenpaare ausgedrückt wird,

- dem intrinsischen Parameter 2, der für die Basenzusammensetzung gemäß der folgenden Gleichung steht:

$$KF[Base] = \frac{0{,}506 * A_{(interessierendes\ Gen)}}{0{,}34} + \frac{0{,}346 * T_{(interessierendes\ Gen)}}{0{,}26} + \frac{0{,}063 * C_{(interessierendes\ Gen)}}{0{,}197} + \frac{0{,}085 * G_{(interessierendes\ Gen)}}{0{,}203}$$

worin $A_{(interessierendes\ Gen)}$, $T_{(interessierendes\ Gen)}$, $C_{(interessierendes\ Gen)}$ und $G_{(interessierendes\ Gen)}$ Brüche sind,

- der experimentelle Parameter für die Multiplikationsrate gemäß der folgenden Gleichung steht:

$$KF[d] = d(interessierendes\ Gen)/d(gfp)$$

worin d die Multiplikationsrate ist, die gemäß der folgenden Gleichung berechnet wird:

$$d = ln(Qf/Qi)/ln2,$$

wobei Qi und Qf jeweils für die Molmengen der Matrix und der Produkte nach Abschluss der PCR stehen.

**19.** Verwendung eines Verfahrens nach irgendeinem der Ansprüche 1 bis 18 zur Kontrolle des Mutationsgrads in einer Bibliothek mutierter Gene, **dadurch gekennzeichnet, dass** es einen zusätzlichen Schritt der Auswahl der Versuchsbedingungen der zufälligen Mutagenese umfasst, die verwendet werden, um Schritt (b) zu implementieren.

**20.** Verwendung eines Verfahrens nach irgendeinem der Ansprüche 1 bis 18, um die Fähigkeit eines Proteins zu untersuchen, Mutationen zu inkorporieren und dabei gleichzeitig eine Aktivität zu erhalten, die das Wildtyp-Protein aufweist.

**21.** Verwendung eines Verfahrens nach irgendeinem der Ansprüche 1 bis 18, um die Fähigkeit eines Proteins zu untersuchen, eine neue Eigenschaft mit einem gegebenen Mutationsgrad zu erwerben.

**22.** Verwendung eines Verfahrens nach irgendeinem der Ansprüche 1 bis 18, um den optimalen Mutationsgrad in den Bibliotheken mutierter interessierender Gene zu bestimmen, um eine Elterngencharge vor der Implementierung von Methoden der molekularen Rekombination zu erzeugen.

**23.** Verwendung eines Verfahrens nach irgendeinem der Ansprüche 1 bis 18, um die Zuverlässigkeit einer der oder der Methoden zur Polymerisation von Nukleinsäuren zu quantifizieren oder zu vergleichen.

**24.** Kit, der in einem Verfahren zur Bestimmung des Mutationsgrads einer Genbibliothek implementiert werden kann, die nach Abschluss der zufälligen Mutagenese eines interessierenden Gens nach irgendeinem der Ansprüche 1 bis 18 oder bei der Verwendung nach den Ansprüchen 19 bis 23 gewonnen wurde, **dadurch gekennzeichnet, dass** er Folgendesumfasst:

- mindestens ein Modellgen, zum Beispiel in Form eines Plasmids;
- mindestens ein Nomogramm, das es ermöglicht, den Mutationsgrad einer durch zufällige Mutagenese des Modellgens gewonnenen Genbibliothek mit der Fraktion der in der Bibliothek beobachteten mutierten Modellgene zu verknüpfen;
- die für die Implementierung der Technik der zufälligen Mutagenese erforderlichen Reagentien, wie zum Beispiel eine thermostabile DNA-Polymerase, dNTP, Magnesium, Mangan, PCR-Puffer, falls die Mutagenesetechnik der fehlerauslösenden PCR entspricht;
- gegebenenfalls die Reagentien, um Mutantenmodellgene zu beobachten;
- eine Gebrauchsanweisung, in der die Korrekturunterfaktoren angegeben sind, die für einen Parameter definiert wurden, der ausgewählt wurde, weil er den Mutationsgrad beeinflusst.

**25.** Kit, der bei einer Verwendung nach Anspruch 19 implementiert werden kann, **dadurch gekennzeichnet, dass** er zusätzlich zu den Bestandteilen des Kits nach Anspruch 24 mindestens ein Nomogramm umfasst, das es ermöglicht, den Mutationsgrad (MG) einer durch zufällige Mutagenese eines Modellgens gewonnenen Genbibliothek mit den Versuchsbedingungen zufälliger Mutagenese zu verknüpfen.

**26.** Kit nach Anspruch 24 oder 25, **dadurch gekennzeichnet, dass** er die Primer für die Amplifikation des Modellgens aufweist, falls die Mutagenesetechnik auf einer Amplifikationstechnik basiert.

**27.** Kit nach irgendeinem der Ansprüche 24 bis 26, **dadurch gekennzeichnet, dass** die bereitgestellten Korrekturunterfaktoren durch Daten vervollständigt werden müssen, wie jene, die insbesondere aus der folgenden Gruppe ausgewählt sind:

- die Länge des interessierenden Gens,
- die Basenzusammensetzung A des interessierenden Gens,
- die Basenzusammensetzung C des interessierenden Gens,
- die Basenzusammensetzung G des interessierenden Gens,
- die Basenzusammensetzung T des interessierenden Gens,
- die Multiplikationszahl der Matrix des Modellgens,
- die Multiplikationszahl der Matrix des interessierenden Gens,

wobei die entsprechenden Werte durch den Forscher in Abhängigkeit von dem interessierenden Gen und dem Ergebnis der zufälligen Mutagenese integriert werden.

**28.** Kit nach irgendeinem der Ansprüche 24 bis 27, **dadurch gekennzeichnet, dass** das Modellgen das von *gfp* ist.

**29.** Kit nach dem vorhergehenden Anspruch, **dadurch gekennzeichnet, dass**, wenn die Mutagenese mit fehlerauslösender PCR durchgeführt wird und das Modellgen *gfp* ist, die verschiedenen Korrekturunterfaktoren ausgewählt sind aus:

- dem intrinsischen Parameter 1, der für die Länge der Gene gemäß der folgenden Gleichung steht:

$$KF[L] = L(interessierendes\ Gen)/717$$

worin L als Anzahl der Basenpaare ausgedrückt wird,

- dem intrinsischen Parameter 2, der für die Basenzusammensetzung auf den Strängen, die die DNA kodieren, gemäß der folgenden Gleichung steht:

$$KF[Base] = \frac{0{,}506 * A_{(interessierendes\ Gen)}}{0{,}34} + \frac{0{,}346 * T_{(interessierendes\ Gen)}}{0{,}26} + \frac{0{,}063 * C_{(interessierendes\ Gen)}}{0{,}197} + \frac{0{,}085 * G_{(interessierendes\ Gen)}}{0{,}203}$$

Worin $A_{(interessierendes\ Gen)}$, $T_{(interessierendes\ Gen)}$, $C_{(interessierendes\ Gen)}$ und $G_{(interessierendes\ Gen)}$ Brüche sind,
- der experimentelle Parameter für die Multiplikationsrate gemäß der folgenden Gleichung steht:

$$KF[d] = d(interessierendes\ Gen)/d(gfp)$$

worin d die Multiplikationsrate ist, die gemäß der folgenden Gleichung berechnet wird:

$$d = \ln(Qf/Qi)/\ln2$$

wobei Qi und Qf jeweils für die Molmengen der Ausgangsmatrix und der Produkte nach Abschluss der PCR stehen.

30. Kit nach irgendeinem der Ansprüche 24 bis 29, **dadurch gekennzeichnet, dass** er mehrere Modellgene und mehrere Nomogramme für jedes Modellgen aufweist, um das Modellgen auszuwählen, das intrinsische Merkmale hat, die dem interessierenden Gen am ähnlichsten sind.

31. Verfahren nach irgendeinem der Ansprüche 24 bis 30, **dadurch gekennzeichnet, dass** die Korrekturunterfaktoren in einer digitalen Datei aufgezeichnet sind, die auf einem geeigneten Träger (CD-Rom, Diskette usw.) gespeichert ist.

32. Kit nach irgendeinem der Ansprüche 24 bis 31, **dadurch gekennzeichnet, dass** das Nomogramm in Form einer digitalen Datenbank aufgezeichnet ist, die auf einem geeigneten Träger gespeichert ist.

33. Verfahren nach irgendeinem der Ansprüche 1 bis 18, **dadurch gekennzeichnet, dass** mindestens einer der Schritte (a), (c) und (d) mit Datenverarbeitungsmitteln implementiert wird.

34. Verfahren nach Anspruch 33, **dadurch gekennzeichnet, dass** die Datenverarbeitungsmittel digitale Daten verarbeiten, die sich auf mindestens eines der folgenden Merkmale beziehen:

- das ausgewählte Modellgen,
- die gewählte Mutagenesetechnik,
- den oder die Parameter, die hinsichtlich ihrer Fähigkeit ausgewählt wurden, den Mutationsgrad einer Bibliothek mutierter Gene bezogen auf eine andere zu beeinflussen, um die Korrekturunterfaktoren zu definieren.

35. Verfahren nach einem der Ansprüche 33 oder 34, **dadurch gekennzeichnet, dass** die Datenverarbeitungsmittel digitale Daten verarbeiten, die sich auf mindestens eines der folgenden Merkmale beziehen:

- die Länge des interessierenden Gens,
- die Basenzusammensetzung A des interessierenden Gens,
- die Basenzusammensetzung C des interessierenden Gens,

- die Basenzusammensetzung G des interessierenden Gens,
- die Basenzusammensetzung T des interessierenden Gens,
- die Multiplikationszahl der Matrix des Modellgens,
- die Multiplikationszahl der Matrix des interessierenden Gens,

wobei die entsprechenden Werte durch den Forscher in Abhängigkeit von dem interessierenden Gen und dem Ergebnis der zufälligen Mutagenese integriert werden.

36. Verfahren nach einem der Ansprüche 33 bis 35, **dadurch gekennzeichnet, dass** die Datenverarbeitungsmittel auf einem vorprogrammierten Rechner oder einem Mikrocomputer verwendet werden.

37. Verfahren nach einem der Ansprüche 33 bis 36, **dadurch gekennzeichnet, dass** es die Datenverarbeitungsmittel ermöglichen, die Daten zu integrieren, die mit den Nomogrammen des oder der Modellgene und mit den Korrekturunterfaktoren verknüpft sind.

38. Vorrichtung, die in einem Verfahren zur Bestimmung des Mutationsgrads einer Bibliothek nach Abschluss der zufälligen Mutagenese eines interessierenden Gens nach irgendeinem der Ansprüche 1 bis 18 und 33 bis 37 oder in eine Verwendung nach den Ansprüchen 19 bis 23 implementiert werden kann, **dadurch gekennzeichnet, dass** sie einen Kit nach einem der Ansprüche 24 bis 32 und einen vorprogrammierten Rechner oder einen Mikrocomputer umfasst.

Figure 1

1   tttcaagagtgccatgcccgagggttatgtacaggaaagaactatattttttcaaagatga 60

61  cgggaactacaagacacgtgctgaagtcaagtttgaaggtgatacccttgttaatagaat 120

121 cgagttaaaaggtgttgatttttaaagaagatggaaacattcttggacacaaattggaata 180

181 caactataactcacacaatgtatacatcatggcagacaaacaaaagaatggaatcaaagc 240

241 taacttcaaagttagacacaacattgaagatggaagcgttcaactggcagaccattatca 300

301 acaaaatactccaattggcgatggccctgtccttttaccagacaaccattacctgtccac 360

361 acaatctgcccttttcgaaagatcccaacgaaaagagagaccacatggtccttcttgagtt 420

421 tgtaacagctgctgggattacacatggcatggatgaactatacaaataaatgagtaaagg 480

481 agaagaactttttcactagagttgtcccaattcttgttgaattagacggtgatgttaatgg 540

541 gcacaaattttctgtcagtggagagggagaaggtgatgcaacatacggaaaacttaccct 600

601 taaatttatttgcactactggaaaactaccagttccgtggccaacacttgtcactactct 660

661 ctcttatggtgttcaatgcttttcgagatacccagatcacatgaaacggcatgactt   717

## Figure 2

**Charge mutationelle en nombre de mutation/ *gfp***

$$y = -0,0083x2 - 0,1111$$
$$R^2 = 0,999$$

Figure 3

|   | A | T | C | G |
|---|---|---|---|---|
| **A** | / | 0,237 | 0,052 | 0,217 |
| **T** | 0,156 | / | 0,151 | 0,039 |
| **C** | 0,024 | 0,033 | / | 0,006 |
| **G** | 0,045 | 0,031 | 0,009 | / |

Figure 4

Matrice cible *gfp*
pour la PCRep

| | |
|---|---|
| A | 0,506 |
| T | 0,346 |
| C | 0,063 |
| G | 0,085 |

Figure 5

Figure 6

**EP 1 774 032 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0009679 A

**Littérature non-brevet citée dans la description**

- **Zaccolo M. et al.** *Journal of Molecular Biology,* 1999, vol. 285, 775-783
- **Cherry J .R. et al.** *Nature Biotechnology,* 1999, vol. 17, 379-384
- **Shafikhani S. et al.** *BioTechniquers,* 1997, vol. 23, 304-310
- **Moore J.C. et al.** *Journal of Molecular Biology,* 1997, vol. 272, 336-347
- **Cariello et al.** *Mutational Research,* 1993, vol. 288, 103-112
- **Brail et al.** *Mutational Research,* 1993, vol. 303, 171-175
- **Greener et al.** *Mol. Biotech.,* 1997, vol. 7, 189-195
- **Cadwell R.C et al.** *PCR Methods Appl.,* 1992, vol. 2, 28-33
- **Leung D.W. et al.** *Techniques,* 1989, vol. 1, 11-15
- **Vartanian J.P. ; Henry M. ; Wain-Hobson S.** Hypermutagenic PCR involving all four transitions and a sizeable proportion of transversions. *Nucleic Acids Res.,* 1996, vol. 24, 2627-2631
- **Wan L. et al.** *Proc. Natl. Acad. Sci. USA,* 1998, vol. 95, 12825-12831

- **You L et al.** *Protein Eng.,* 1996, vol. 9, 77-83
- **Miura T. et al.** *J. theor. Biol.,* 2001, vol. 209, 497-502
- **Stemmer W.** *Nature,* 1994, vol. 370, 389-391
- **Zhao H. et al.** *Nature Biotechnology,* 1998, vol. 16, 258-261
- **Coco W. et al.** *Nature Biotechnology,* 2001, vol. 19, 354-359
- **Pompon D. et al.** *Gene,* 1989, vol. 83, 15-24
- **Volkov A. et al.** *Nucleic Acids Research,* 1999, vol. 27, 18
- **Cadwell R.C. ; Joyce G.F.** *PCR Methods Appl.,* 1992, vol. 2, 28-33
- **Sambrook J. et al.** Molecular cloning: a laboratory manual. Cold Spring Harbor Laboratory Press, 1989
- **Daugherty P et al.** *Proc. Natl. Acad. Sci. USA,* 2000, vol. 97, 2029-2034
- **Shafikhani S. et al.** *BioTechniques,* 1997, vol. 23, 304-310
- **Daugherty P. et al.** *Proc. Natl. Acad. Sci. USA,* 2000, vol. 97, 2029-2034